**Europäisches Patentamt**

**European Patent Office**

**Office européen des brevets**

(19)

(11) Numéro de publication: **0 022 698**
**B1**

(12) **FASCICULE DE BREVET EUROPÉEN**

(45) Date de publication du fascicule du brevet:
**13.08.86**

(51) Int. Cl.⁴: **G 01 N 33/554**

(21) Numéro de dépôt: **80400959.5**

(22) Date de dépôt: **26.06.80**

(54) Réactif de diagnostic des parasitoses et des allergies, procédé de diagnostic à l'aide dudit réactif et trousse de réactif pour ledit procédé.

(30) Priorité: **28.06.79 FR 7916772**
**27.05.80 FR 8011689**

(43) Date de publication de la demande:
**21.01.81 Bulletin 81/3**

(45) Mention de la délivrance du brevet:
**13.08.86 Bulletin 86/33**

(84) Etats contractants désignés:
**BE CH DE IT LI LU NL SE**

(73) Titulaire: **INSTITUT PASTEUR, 25- 28, rue du Docteur Roux, F-75724 Paris Cédex 15 (FR)**

(72) Inventeur: **Leynadier, Francisque, 42, rue de Chaligny, F-75012 Paris (FR)**
Inventeur: **Luce, Hervé, 54, rue de Picpus, F-75012 Paris (FR)**

(74) Mandataire: **Ores, Irène, CABINET ORES 6, Avenue de Messine, F-75008 Paris (FR)**

(56) Documents cité:
FR-A-2 315 251

CHEMICAL ABSTRACTS, vol. 88, no. 19, 8 mai 1978, page 369, abrégé 134624g, Columbus, Ohio, USA, J. BENVENISTE "The in-vitro basophil degranulation test"
THE JOURNAL OF IMMUNOLOGY, vol. 118, no. 4, avril 1977, USA, M.C. CONROY et al. "Measurement of IgE on human basophils: relation to serum IgE and anti-IgE-induced histamine release", pages 1317-1321
CHEMICAL ABSTRACTS, vol. 79, no. 3, 23 juillet 1973, page 77, abrégé 14059u, Columbus, Ohio, USA, A. CHAGNON et al. "Use of an organic buffer (HEPES) (N-2-hydroxyethylpiperazine-N'-2-ethanesulfonic acid) in human lymphocytoid cell line cultures"

(56) Documents cité: (suite)
CHEMICAL ABSTRACTS, vol. 91, no. 5, 30 juillet 1979, page 263, abrégé 35114w, Columbus, USA, VEGA, C.A. et al. "Standardization of biological buffers in waterdimethyl sulfoxide mixtures"
CHEMICAL ABSTRACTS, vol. 89, no. 13, 25 septembre 1978, page 604, abrégé 105681p, Columbus, Ohio, USA, MALVEAUX, F.J. et al. "IgE receptors on human basophils. Relationship to serum IgE concentration"

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# 0 022 698

**Description**

La présente invention est relative à un nouveau réactif de diagnostic des parasitoses et des allergies, à un procédé de diagnostic des parasitoses et des allergies, notamment des parasitoses à vers qui provoquent une augmentation des immunoglobulines spécifiques circulantes telles qu' IgE, $IgG_4$, IgM, à l'aide de ce réactif et à un kit prêt à l'emploi pour la mise en oeuvre dudit procédé de diagnostic.

La protection de l'homme et des animaux domestiques contre les affections parasitaires représente l'un des grands problèmes mondiaux en matière de santé publique. Si les pays développés ont résolu un certain nombre de leurs problèmes parasitologiques sans les avoir cependant tous supprimés, les parasitoses constituent l'obstacle majeur au développement économique de beaucoup de pays, et en particulier des pays des régions chaudes du globe, qui sont celles qui comptent la plus grande prolifération humaine.

Il est essentiel pour pouvoir entreprendre une lutte efficace contre le fléau que représentent les parasitoses, de pouvoir réaliser un diagnostic sûr, fiable et précoce des parasitoses.

Si les examens cliniques, les examens radiologiques et les renseignements tirés de l'anamnèse sont d'un grand secours pour l'établissement du diagnostic, c'est essentiellement grâce aux méthodes biologiques et notamment immunoparasitologiques que les techniques d'investigation ont profondément évolué. C'est grâce, essentiellement, à l'étude de l'hémogramme et des examens séro-immunologiques que l'on dispose actuellement, dans le domaine de la parasitologie, des "arguments d'orientation" et des "arguments de certitude" (Gentilini et Pinon, Vie Médicale, 7 Février 1973).

Il est fréquent actuellement de rapporter à une étiolologie parasitaire une manifestation fébrile jadis jugée "hépatique", "urinaire", ou "allergique".

De nombreuses techniques sérologiques ont été ainsi proposées: des tests cutanés par intradermoréaction, des tests d'agglutination des particules de latex et des hématies sensibilisées, des tests d'hémagglutination, des réactions de précipitation en gélose, des analyses immuno-électrophorétiques, des tests par immunofluorescence, des réactions de fixation du complément, etc... Ces méthodes désormais classiques de diagnostic ont pu se développer ces derniers temps, depuis que l'on dispose de fractions antigéniques parfaitement connues et individualisées, et depuis également, que l'on a maîtrisé la conservation et la stabilisation de ces antigènes, ainsi que leur standardisation. Toutes ces méthodes ont leurs avantages et leurs inconvénients, aucune n'est infaillible. C'est ainsi que A. Capron et Collab. (Path. Biol. 1970, Vol. 18, pages 357—365) comparent les résultats obtenus pour le diagnostic immunologique de l'échinococcose humaine par les quatre méthodes suivantes:

1) l'immunoélectrophorèse (I.E.),
2) l'hémagglutination (H.M.G.),
3) la réaction d'agglutination au latex (A.L.), et
4) la réaction de fixation du complément (R.F.C).

Les résultats globaux du diagnostic immunologique de l'hydratidose obtenus par ces Auteurs, sont résumés dans le Tableau I ci-après.

TABLEAU I

| Méthode | Nombre de malades étudiés | Nombre de malades positifs | Pourcentage de positivité | Minimum significatif observé | Maximum | Moyenne |
|---|---|---|---|---|---|---|
| I.E. | 350 | 321 | 91,7 % | 1 | 18 | 4,8 |
| R.F.C. | 318 | 227 | 71,4 % | 1/2 | 1/256 | 1/20 |
| H.M.G. | 330 | 260 | 78,8 % | 1/512 | 1/512000 | 1/30000 |
| A.L. | 279 | 220 | 78,8 % | 1/2 | 1/512 | 1/50 |

Ce Tableau met en évidence les pourcentages de positivité, ainsi que les limites inférieures et supérieures obtenues par les diverses méthodes utilisées. On remarque que l'immunoélectrophorèse a permis de déceler 91,7 % des cas. Dans tous les cas où il a été conclu positivement à l'existence d'une hydatidose, celle-ci a été confirmée par la voie opératoire, et il n'a jamais été posé de diagnostic immunologique par excès. Par contre, A. Capron et Collab. ont noté qu'un certain nombre de malades pour lesquels la réponse immunologique a été négative, étaient bien porteurs d'une hydatidose. Le pourcentage d'échec était de 15,4 % pour I.E., 29 % pour R.F.C., 21,4 % pour H.M.G. et 24 % pour A.L.

Une autre méthode d'immunodiagnostic proposée, a trait à la technique de radioallergosorption ("radioallergosorbent-test" des anglo-saxons ou Rast). Ce test est basé sur le fiat quee les sujets atteints de

2

parasitose ont des taux d'immunoglobulines E (IgE) spécifiques anormalement élevés. La méthode a été décrite en détail par Ceska et Collab. (J. Allergy Clin. Immunol. 1972, 49, 1). C'est ainsi que D. Vervloet et Collab. [Rev. franç. Allergol. 1976, 16 (No. 2), pages 73—78] ont utilisé cette technique du Rast chez 60 sujets présentant une hydatidose confirmée chirurgicalement et ont pu mettre en évidence 54 fois, soit dans 90 % des cas, la présence d'IgE spécifiques à l'égard des antigènes solubles du kyste hydatique. Ils ont également mis en évidence une bonne corrélation entre le taux d'IgE spécifiques mesuré par le Rast et le taux d'IgE totales. Mais, par contre, ils n'ont pas trouvé de corrélation entre le taux des anticorps IgE et le taux des anticorps mesurés par les techniques classiques d'immuno-électrophorèse, de fixation du complément, d'immunofluorescence et d'hémagglutination. Ces Auteurs, comme également, par exemple Niklaus Weiss et Collab. ("The Lancet", 9 Décembre 1978) qui ont utilisé le Rast chez des sujets infestés par Schistosoma haematobium et S. mansoni, ou Dessaint et Collab. (Immunol. 1975, 29, 813) qui ont dosé les IgE spécifiques chez des sujets atteints d'hydatidose et de bilharziose, concluent que le Rast peut s'ajouter à la "batterie classique" des tests immunologiques, sans les supplanter, en raison de sa fiabilité insuffisante et du fait qu'il ne s'agit pas d'un test quantitatif, mais d'un test uniquement qualitatif. Il y a lieu également de citer les tests de dosage de l'histamine plus fiables que le Rast.

Un test de dégranulation des basophiles a, par ailleurs, été décrit dans la littérature (cf. "Chemical Abstracts Vol. 88, No. 19, 8 mai 1978, page 369); il s'agit d'un test in vitro en présence d'antigène, en vue de son application au diagnostic des allergies; la réaction a lieu à une température maximum de 37°C, au bout de 10 minutes, et elle nécessite la présence de calcium; elle est progressivement inhibée par du plasma de lapin recevant >1 d'injection d'antigène. Cependant, cette publication ne précise pas comment le réactif de diagnostic est obtenu et le déroulement du test n'est pas décrit avec précision puisque seules sont indiquées une température, une durée et la nécessité de la présence de calcium.

Un article paru dans "The Journal of Immunology" Vol. 118, No. 4, avril 1977 sous la signature de Conroy, Adkinson et Lichtenstein, Utilise pour la mesure des IgE sur des basophiles humains une source de leucocytes provenant non pas de sang total, mais de plasma, les préparations de leucocytes obtenues contenant moins de 1% de basophiles, c'est-à-dire qu'elles ne sont pas enrichies en basophiles. Au demeurant, cette publication antérieure, qui ne propose pas une suspension concentrée et enrichie en basophiles, vise à tester la sensibilité de cellules basophiles auxquelles sont fixées des IgE, à des anti-IgE pour déterminer la relation entre les IgE fixées aux cellules et la concentration d'anti-IgE requise pour libérer de l'histamine. Ni le but recherché dans cette publication, ni les moyens mis en oeuvre (fraction de départ différente et produit obtenu différent puisqu'il ne s'agit pas d'une suspension enrichie en basophiles) n'entrent pas, toutefois, dans le cadre de la présente invention, qui vise à un procédé de diagnostic de parasitoses et d'allergies à l'aide d'un nouveau réactif constitué par une suspension enrichie en basophiles.

Dans le domaine de l'allergologie, les Inventeurs, en se basant sur les travaux de Shelley [notamment dans "Nature", 1962, 195, p. 1181—1183; "Blood", 1962, 19, p. 208—216; Trans. Stud. Coll. Physns Philad., 1964, 32, p. 15—19], repris ultérieurement par Hirsch et Zastrow (J. Allergy Clin. Immunol. 1972, 50, p. 338—347) et Soifer et Hirsch [J. Allergy Clin. Immunol., 1975, 56, p. 127—132] ont appliqué le test de dégranulation des polynucléaires basophiles humains avec une précision du diagnostic de l'ordre de 94 %, en utilisant une suspension leucocytaire enrichie 10 à 20 fois en polynucléaires basophiles (par rapport au sang circulant), dont les cellules sont fixées sur lame et colorées au bleu de toluidine [cf. F. Leynadier, H. Luce et J. Dry, Rev. Franç. d'Allergol., 1977, 17 (No. 4), p. 215—218].

En partant de la connaissance du fait que la production de quantités importantes d'IgE spécifiques circulantes est l'une des caractéristiques de la réponse immune à la plupart des parasitoses à vers, les Inventeurs ont cherché à adapter la méthode de mesure des IgE par des antigènes spécifiques en utilisant des suspensions enrichies en polynucléaires basophiles porteurs d'IgE, mises sur lames, qu'ils avaient précédemment mise au point dans le domaine de l'allergologie, au diagnostic de diverses parasitoses à vers.

La présente invention a en conséquence pour but de pourvoir à un nouveau réactif de diagnostic des parasitoses et des allergies, et notamment des parasitoses à vers qui provoquent une augmentation des immunoglobulines spécifiques circulantes fixées sur les basophiles, telles que les IgE en particulier, lequel réactif lorsqu'il est mis en oeuvre dans le procédé de diagnostic qui constitue également l'un des buts de l'invention, permit d'obtenir des diagnostics extrêmement fiables tout en étant relativement simple à réaliser, tandis que les techniques qui permettent d'obtenir ce réactif, lui confèrent des concentrations en basophiles suffisamment élevées pour que les résultats obtenus en présence d'antigène, au cours du diagnostic, soient statistiquement interprétables en toute sécurité.

La présente invention a pour objet un procédé de diagnostic des parasitoses et des allergies, et en particulier des parasitoses à vers et des allergies qui provoquent une augmentation des immunoglobulines spécifiques circulantes, et notamment des IgE, qui consiste:— à mettre en contact pendant environ 15 minutes à 37°C, un réactif de diagnostic des parasitoses et des allergies constitué par une suspension de polynucléaires basophiles porteurs d'IgE spécifiques, dans un tampon approprié, avec de l'antigène spécifique de la parasitose ou de l'allergie que l'on cherche à diagnostiquer, contenu à différentes concentrations dans un certain nombre de puits d'une lame ou d'une boîte de lecture de diagnostic, tandis que les puits restants de ladite lame ou boîte contiennent le réactif de diagnostic seul, pour servir de témoins; — à procéder à un séchage rapide de la lame ou boîte ainsi préparée; — à procéder à la fixation et à la coloration rapides de la lame préparée de la sorte, à l'aide de moyens de fixation et de coloration rapides qui colorent sélectivement les granules cytoplasmiques des basophiles; — à compter les polynucléaires basophiles (PB) au microscope optique, avec un grossissement approprié, respectivement dans les puits témoins et dans les différents puits contenant le réactif et différentes concentrations d'antigène; — et à établir par comparaison de la diminution du nombre de polynucléaires basophiles dans les puits contenant l'antigène à diverses concentrations, par rapport au nombre de polynucléaires basophiles contenus dans les puits témoins, l'index de dégranulation:

$$ID = \frac{PB\ témoins\ —\ PB\ antigène}{PB\ témoins} \times 100$$

ou pourcentage de basophiles qui ont apparemment disparu en présence de l'antigène, qui, s'il est supérieur à 35 %, permet de diagnostiquer la présence de la parasitose ou de l'allergie recherchée, lequel procédé est caractérisé en ce que le réactif de diagnostic introduit dans les puits de la lame ou de la boîte susdite, est constitué par une suspension enrichie en polynucléaires basophiles, dans un tampon non destructeur vis-à-vis des cellules basophiles, tel que le tampon Hépès CM ou le tampon Pipès, notamment, laquelle suspension contient de 300 à 1000 basophiles par µl et a été obtenu à partir d'un échantillon de sang prélevé chez un patient humain ou animal supposé atteint de la parasitose ou de l'allergie à diagnostiquer, lequel échantillon de sang a été soumis à l'un ou l'autre des traitements suivants:

— ou bien l'échantillon de sang est soumis à une centrifugation, pendant une durée et à une vitesse appropriées, pour recueillir une couche leucocytaire et un plasma surnageant riche en plaquettes, lequel a été rejeté, tandis que la couche leucocytaire recueillie a été mélangée avec une quantité appropriée d'un tampon non destructeur à l'égard des cellules basophiles tel que le tampon Hépès CM ou le tampon Pipès, notamment;

— ou bien ledit échantillon de sang total est mélangé avec un sel de l'acide éthylènediaminetétraacétique, puis mélangé avec un tampon Hépès de composition appropriée;

— ou bien ledit échantillon de sang total est mélangé avec un sel de l'acide éthylènediaminetétraacétique avantageusement sous la forme de l'un de ses sels, et de préférence sous la forme de son sel potassium (EDTA $K_3$), puis mélangé avec un tampon Hépès de composition appropriée contenant de l'héparine à 5—20 unités/ml;

— après quoi, un liquide de densité 1,079—1,085 dont l'osmolarité est comprise entre 280 et 320 milliosmoles, avantageusement constitué par une composition comprenant du métrizoate de sodium et du Ficoll ou du Percol, est injecté sous le mélange de couche leucocytaire et de tampon ou sous le mélange de sang total prélevé sur sel d'acide éthylènediaminetétraacétique, avec un tampon éventuellement hépariné,

— le mélange obtenu étant centrifugé avec ledit liquide de densité pour obtenir un anneau contenant une concentration élevée en basophiles,

— lequel anneau est prélevé, puis remis en suspension dans un tampon tel que le tampon susdit; ledit procédé étant, en outre, caractérisé en ce que les moyens de fixation et de coloration rapides susdits sont constitués par une composition unique qui réalise simultanément la fixation et la coloration rapides et qui comprend un colorant métachromatique, qui est de préférence du bleu de toluidine, associé à du sulfate d'aluminium, à un halogénure de cétylpyridinium et:

— soit à du glutaraldéhyde et à du formaldéhyde, en solution dans un mélange d'éthanol et de méthanol absolus et d'eau, laquelle solution a un pH acide inférieur à 2,5;

— soit à du méthanol éventuellement associé à du formaldéhyde et/ou à de l'éthanol, en l'absence de glutaraldéhyde;

— soit à du propylèneglycol associé à du méthanol, en l'absence de glutaraldéhyde, de formaldéhyde et d'éthanol.

Conformément à la présente invention, le réactif de fixation et de coloration rapides, simultanées, nécessaire à la mise en oeuvre du procédé de diagnostic défini dans ce qui précède, est constitué par une solution hydroalcoolique à pH acide, inférieur à 2,5, caractérisée en ce qu'elle présente la composition suivante:

4

| | |
|---|---|
| Bleu de toluidine ou colorant métachromatique analogue | 2,2 g/litre |
| Sulfate d'aluminium | 7,5 g/litre |
| Halogénure de cétylpyridinium | 0,75 g/litre |
| Glutaraldéhyde | 20 g/litre |
| Formaldéhyde | 35 g/litre |
| Méthanol absolu | 300 ml/litre |
| Ethanol absolu | 300 ml/litre |
| Eau        q.s.p. | 1000 ml |

Selon un mode de réalisation avantageux du réactif de fixation et de coloration rapides, simultanées, mis en oeuvre dans le procédé de diagnostic conforme à l'invention, la solution hydroalcoolique susdite présente la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Glutaraldéhyde | 1,5 à 2,5 % |
| Formaldéhyde à 30 % | 270 à 330 ml |
| Méthanol | 270 à 330 ml |
| Ethanol | 270 à 330 ml |
| Eau        q.s.p. | 1000 ml |

Selon un autre mode de réalisation avantageux de ces solutions de fixation et de coloration rapides, simultanées, celles-ci présentent la composition qualitative et quantitative suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Méthanol | 300 ml±10 % |
| Formaldéhyde à 30 % | 300 ml±10 % |
| Ethanol | 300 ml±10 % |
| Eau        q.s.p. | 1000 ml |

Selon encore un autre mode de réalisation de ces solutions de fixation et de coloration rapides, simultanées, celles-ci présentent la composition qualitative et quantitative suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Méthanol | 830 à 990 ml |
| Eau        q.s.p. | 1000 ml |

5

Selon encore un autre mode de réalisation de ces solutions de fixation et de coloration rapides, simultanées, celles-ci présentent la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Propylèneglycol | 250 ml à 600 ml ± 10 % |
| Méthanol | 200 ml à 750 ml ± 10 % |
| Eau          q.s.p. | 1000 ml |

Conformément à la présente invention, l'halogénure de cétylpyridinium mis en oeuvre, est le chlorure ou le bromure de cétylpyridinium.

La présente invention a de plus pour objet, un ensemble prêt à l'emploi, ou kit, de préférence contenu dans un coffret unique, pour la réalisation du test de diagnostic des parasitoses et des allergies, conforme à la présente invention, lequel ensemble est caractérisé en ce qu'il comprend, en combinaison:— une pluralité de lames, boîtes ou analogues présentant chacune un certain nombre de puits de diamètre égal dont la majeur partie, à l'exception des puits devant constituer les témoins, contiennent une quantité prédéterminée d'un antigène spécifique d'une parasitose ou d'une allergie à diagnostiquer, lequel antigène est introduit à l'état sec, de préférence lyophilisé, dans chacun des puits, lesdites quantités préterminées variant d'une colonne de puits à la suivante et lesdits puits étant destinés à recevoir, pour la réalisation du test de diagnostic conforme à l'invention, une quantité appropriée du réactif de diagnostic conforme à l'invention; — une pluralité de tubes contenant des quantités prédosées d'un tampon approprié tel que tampon Hépès CM ou Pipès; une pluralité de récipients adaptés tels que des ampoules, des tubes, des seringues ou analogues, contenant chacun un liquide de densité 1,079—1,085; — au moins un récipient contenant une solution hydroalcoolique de fixation et de coloration rapides, simultanées, des lames, boîtes ou analogues susdites, conforme à l'invention.

Selon un mode de réalisation avantageux de l'ensemble prêt à l'emploi conforme à la présente invention:— chacun des puits contenant un antigène spécifique de la parasitose ou de l'allergie à diagnostiquer contient 10 à 20 µl dudit antigène à l'état lyophilisé et est propre à recevoir, lors de la réalisation du test de diagnostic conforme à l'invention, des quantités variables croissantes, comprises, de préférence, entre 3 et 20 µl, du réactif de diagnostic conforme à l'invention; — chaque tube contenant une quantité prédosée de tampon contient 5 ml dudit tampon; — chaque ampoule, tube, seringue ou analogue, contenant du liquide de densité contient 5 ml dudit liquide de densité 1,079—1,085; — le ou chaque récipient contenant une solution hydroalcoolique de fixation et de coloration rapides, simultanées, des lames, boîtes ou analogues préparées, renferme une solution à pH inférieur à 2,5, présentant la composition suivante:

| | |
|---|---|
| Bleu de toluidine ou colorant métachromatique analogue | 2,2 g/litre environ |
| Sulfate d'aluminium | 7,5 g/litre environ |
| Halogénure de cétylpyridinium | 0,75 g/litre environ |
| Glutaraldéhyde | 20 g/litre environ |
| Formaldéhyde | 35 g/litre environ |
| Méthanol absolu | 300 ml/litre environ |
| Ethanol absolu | 300 ml/litre environ |
| Eau          q.s.p. | 1000 ml |

Conformément à l'invention, les lames, boîtes ou analogues de lecture de diagnostic mises en oeuvre, sont choisies notamment parmi:— les lames présentant une pluralité de puits de diamètre égal, constitués par des cercles de paraffine; — les lames sur lesquelles est collé un ruban adhésif préalablement percé d'une pluralité de trous de diamètre égal qui constituent les puits; — des boîtes de plastique transparent au fond desquelles est collé un ruban adhésif percé d'une pluralité de trous comme indiqué ci-dessus.

Selon un mode de réalisation avantageux du kit ou ensemble prêt à l'emploi pour la réalisation du test de diagnostic conforme à la présente invention, le tampon Hépès contenu en quantités prédosées dans des ampoules ou des tubes appropriés comprend de 4 à 25 ml d'une solution molaire de tampon Hépès, 0,932 ml de KCl à 10 %, 0,550 ml de CaCl$_2$ à 10 %, 0,510 ml de MgCl$_2$ à 10 %, de 850 à 900 ml de NaCl à 9 %, en solution dans une quantité d'eau suffisante pour porter la solution à 1000 ml, ou/et la solution de fixation et de coloration rapides, simultanées, des préparations antigéniques (ou témoins) portées par les lames de lecture de diagnostic, contenue dans le récipient correspondant, présente la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Glutaraldéhyde | 1,5 à 2,5 % |
| Formaldéhyde à 30 % | 300 ml±10 % |
| Méthanol | 300 ml±10 % |
| Ethanol | 300 ml±10 % |
| Eau          q.s.p. | 1000 ml |

ou la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Formaldéhyde à 30 % | 300 ml±10 % |
| Méthanol | 300 ml±10 % |
| Ethanol | 300 ml±10 % |
| Eau          q.s.p. | 1000 ml |

ou bien la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Méthanol | 830 à 990 ml |
| Eau          q.s.p. | 1000 ml |

on encore la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Propylèneglycol | 250 ml à 600 ml±10 % |
| Méthanol | 200 ml à 750 ml±10 % |
| Eau          q.s.p. | 1000 ml |

Selon un mode de réalisation avantageux du kit prêt à l'emploi conforme à la présente invention, pour

**0 022 698**

la réalisation du test de diagnostic, celui-ci se compose d'un coffret qui comprend:— une pluralité de récipients adaptés tels que des ampoules ou des tubes contenant des quantités prédosées d'un tampon Hépès approprié, et notamment du tampon Hépès tel que défini plus haut, — une pluralité d'ampoules, de tubes ou de seringues, contenant chacun des quantités prédosées d'un liquide de densité 1,079—1,085, — une pluralité de lames ou de boîtes de lecture de diagnostic présentant chacune un certain nombre de puits, dont la majeure partie, à l'exception des puits témoins, sont destinés à recevoir des quantités prédéterminées variables d'un antigène spécifique d'une allergie ou d'une parasitose à diagnostiquer, ainsi que des quantités prédéterminées variables du réactif de diagnostic conforme à l'invention, — et une pluralité d'ampoules ou de tubes, renfermant des quantités prédosées d'une solution de fixation et de coloration rapides, simultanées, du type des solutions définies ci-dessus.

Selon un autre mode de réalisation avantageux du kit prêt à l'emploi conforme à la présente invention, celui-ci se compose de deux coffrets séparés dont l'un, qui est destiné à la séparation des cellules basophiles, comprend:— une pluralité de récipients adaptés, tels que des tubes ou des ampoules contenant des quantités prédosées d'un tampon approprié, et notamment du tampon Hépès tel que défini plus haut, — une pluralité d'ampoules ou de tubes contenant chacun des quantités prédosées d'un liquide de densité 1,079—1,085; et dont l'autre coffret comprend:— une pluralité de lames ou boîtes de lecture de diagnostic présentant chacune un certain nombre de puits, dont la majeure partie à l'exception des puits témoins, sont destinés à recevoir des quantités prédéterminées variables d'un antigène spécifique d'une allergie ou d'une parasitose à diagnostiquer, ainsi que des quantités prédéterminées variables du réactif de diagnostic conforme à l'invention, — et une pluralité d'ampoules ou de tubes renfermant des quantités prédosées d'une solution de fixation et de coloration rapides, simultanées, du type des solutions définies ci-dessus, pour la réalisation du test de diagnostic proprement dit.

Selon encore un autre mode de réalisation avantageux du kit prêt à l'emploi en un coffret ou en deux coffrets, conforme à la présente invention, le tampon Hépès contenu en quantités prédosées dans une pluralité de récipients adaptés, tels que des tubes ou des ampoules, présente la composition suivante:

| | |
|---|---|
| Tampon Hépès, solution molaire | 4 à 25 ml |
| KCl 10 % | 0,932 ml |
| $CaCl_2$ 10 % | 0,550 ml |
| $MgCl_2$ 10 % | 0,510 ml |
| NaCl 9 ‰ | 850 à 900 ml |
| Héparine | 10 à 15 unités/ml |
| Eau          q.s.p. | 1000 ml |
| pH | 7,4—7,6 |

Selon un autre mode de réalisation avantageux du kit prêt à l'emploi en un coffret ou en deux coffrets, conforme à la présente invention, le tampon Hépès contenu en quantités prédosées dans une pluralité de récipients adaptés, tels que des tubes ou des ampoules, présente la composition suivante:

| | |
|---|---|
| Tampon Hépès, solution molaire | 4 à 25 ml |
| KCl 10 % | 0,932 à 1,2 ml |
| NaCl 9 ‰ | 850 à 900 ml |
| Eau          q.s.p. | 1000 ml |
| pH | 7,4—7,6 |

auquel cas, les lames ou les boîtes pour la lecture du diagnostic comportent, dans chacun de leurs puits destinés à recevoir de l'antigène, une quantité prédéterminée de calcium et de magnésium sous la forme de leurs sels, et notamment de leurs chlorures.

Selon une disposition avantageuse de ce mode de réalisation, le calcium est présent à raison d'environ 1 mmole/litre, et le magnésium est présent à raison d'environ 1 mmole/litre.

La présente invention a également pour objet un réactif de diagnostic des parasitoses et des allergies, et notamment des parasitoses à vers que provoquent une augmentation des immunoglobulines

8

spécifiques circulantes, et notamment des IgE, caractérisé en ce qu'il est constitué par une suspension enrichie en polynucléaires basophiles porteurs d'IGE spécifiques, qui contient de 300 à 1000 basophiles par µl et que est obtenue à partir d'un échantillon de sang prélevé chez un patient humain ou animal chez lequel on recherche une parasitose ou une allergie, lequel échantillon de sang a été soumis à l'un ou l'autre des traitements suivants:— ou bien l'échantillon de sang est soumis à une centrifugation, pendant une durée et à une vitesse appropriées, pour recueillir une couche leucocytaire et un plasma surnageant riche en plaquettes, lequel a été rejeté, tandis que la couche leucocytaire recueillie a été mélangée avec une quantité appropriée d'un tampon non destructeur à l'égard des cellules basophiles tel que le tampon Hépès CM ou le tampon Pipès, notamment;

— ou bien ledit échantillon de sang total est mélangé avec un sel de l'acide éthylènediaminetétraacétique, puis mélangé avec un tampon Hépès de composition appropriée;

— ou bien ledit échantillon de sang total est mélangé avec un sel de l'acide éthylènediaminetétraacétique avantageusement sous la forme de l'un de ses sels, et de préférence sous la forme de son sel de potassium (EDTA $K_3$), puis mélangé avec un tampon Hépès de composition appropriée contenant de l'héparine à 5—20 unités/ml;

— après quoi, un liquide de densité 1,079—1,085 dont l'osmolarité est comprise entre 280 et 320 milliosmoles, avantageusement constitué par une composition comprenant du métrizoate de sodium et du Ficoll ou du Percol, est injecté sous le mélange de couche leucocytaire et de tampon ou sous le mélange de sang total prélevé sur sel d'acide éthylènediaminetétraacétique, avec un tampon éventuellement hépariné,

— le mélange obtenu étant centrifugé avec ledit liquide de densité pour obtenir un anneau contenant une concentration élevée en basophiles,

— lequel anneau est prélevé, puis remis en suspension dans un tampon tel que le tampon susdit,

— la suspension ainsi formée étant prête à être déposée dans les puits d'une lame ou d'une boîte de lecture de diagnostic.

Conformément à la présente invention, le tampon Hépès non destructeur à l'égard des cellules basophiles, auquel est mélangée la couche leucocytaire obtenue après centrifugation du mélange sous lequel a été injecté le liquide de densité susdit, est un tampon qui présente l'une des compositions suivantes:

|   |     |                                 |          |
|---|-----|---------------------------------|----------|
|   | 1)  | Tampon Hépès, solution molaire  | 25 ml    |
|   |     | KCl 10 %                        | 0,932 ml |
|   |     | CaCl$_2$ 10 %                   | 0,550 ml |
|   |     | MgCl$_2$ 10 %                   | 0,510 ml |
|   |     | NaCl 9 ‰ (7,6 g)                | 844,11 ml |
|   |     | H$_2$O       q.s.p.             | 1000 ml  |
|   | 2)  | Tampon Hépès, solution molaire  | 4 à 25 ml |
|   |     | KCl 10 %                        | 0,932 ml |
|   |     | CaCl$_2$ 10 %                   | 0,552 ml |
|   |     | MgCl$_2$ 10 %                   | 0,510 ml |
|   |     | NaCl 9 ‰                        | 850 à 900 ml |
|   |     | Eau       q.s.p.                | 1000 ml  |
|   |     | pH                              | 7,4—7,6  |

| | | |
|---|---|---|
| 3) | Tampon Hépès, solution molaire | 4 à 25 ml |
| | KCl 10 % | 0,932 ml |
| | CaCl$_2$ 10 % | 0,550 ml |
| | MgCl$_2$ 10 % | 0,510 ml |
| | NaCl 9 ‰ | 850 à 900 ml |
| | Héparine | 10 à 15 unités/ml |
| | Eau        q.s.p. | 1000 ml |
| | pH | 7,4—7,6 |
| 4) | Tampon Hépès, solution molaire | 4 à 25 ml |
| | KCl 10 % | 0,932 ml |
| | NaCl 9 ‰ | 850 à 900 ml |
| | Eau        q.s.p. | 1000 ml |
| | pH | 7,4—7,6 |

Outre les dispositions qui précèdent, l'invention comprend encore d'autres dispositions, qui ressortiront de la description qui va suivre.

L'invention vise plus particulièrement les nouveaux réactifs de diagnostic des parasitoses et des allergies, ainsi que leur procédé de préparation, conformes aux dispositions qui précèdent, et les procédés de diagnostic mettant en oeuvre lesdits réactifs, ainsi que les moyens mis en oeuvre pour réaliser lesdits procédés de diagnostic.

L'invention sera mieux comprise à l'aide du complément de description qui va suivre, qui se réfère à des exemples de préparation des réactifs de diagnostic conformes à la présente invention, à des exemples des tampons utilisables pour préparer lesdits réactifs, à des exemples de solutions de fixation et de coloration rapides, simultanées, utilisables dans le procédé de diagnostic conforme à l'invention, ainsi qu'à un test de diagnostic à l'aide dudit réactif, les résultats de ce test étant illustrées par des graphiques et par la représentation de lames de lecture de diagnostic, dans les dessins annexés.

Il doit être bien entendu toutefois, que ces exemples et dessins sont donnés uniquement à titre d'illustration de l'objet de l'invention, dont ils ne constituent en aucune manière une limitation.

I—Exemples de preparation d'un reactif de diagnostic de parasitose ou d'allergie, conforme a l'invention
Exemple 1

a) On prélève 5 à 10 cm$^3$ d'un échantillon de sang total chez un patient supposé atteint de la parasitose ou de l'allergie que l'on vise à diagnostiquer, le prélèvement étant effectué de préférence à jeûn sur une quantité strictement calculée d'héparine (10 à 15 unités par ml).

b) Si le sang n'est pas traité immédiatement, il peut être placé au réfrigérateur à une température ne dépassant pas 4°C, et pendant une durée ne dépassant pas 3 à 4 heures.

Le sang total prélevé est centrifugé à 130 g à 150 g durant 15 minutes, afin de séparer le plasma contenant les plaquettes, d'une couche leucocytaire, qui contient également les hématies.

c) On prélève et on jette le plasma surnageant contenant les plaquettes, jusqu'à environ 2 à 4 mm de la couche leucocytaire, cette opération pouvant avantageusement être effectuée à l'aide d'une seringue munie d'une aiguille longue, d'un cathéter ou d'une pipette Pasteur.

On récupère d'autre part, la couche leucocytaire et environ 1 cm$^3$ d'hématies sous-jacentes.

d) Dans un tube, de 100×20 par exemple, dans lequel on a déposé 5 ml de tampon Hépès CM qui présente la composition suivante:

| | |
|---|---|
| Tampon Hépès, solution molaire | 25 ml |
| KCl 10 % | 0,932 ml |
| CaCl$_2$ 10 % | 0,550 ml |
| MgCl$_2$ 10 % | 0,510 ml |
| NaCl 9 ‰          (7,6 g) | 844,11 ml |
| H$_2$O distillée          q.s.p. | 1000 ml |

que l'on ajuste avec une dizaine de gouttes de NaOH N à pH entre 7,4 et 7,6;
et dont on vérifie que l'osmolarité est entre 290 et 300 mOsM; pour 25 mmoles de tampon, on introduit la couche leucocytaire et le cm$^3$ d'hématies récupérées, qui l'on mélange avec le tampon.

e) A l'aide d'une seringue, de préférence munie d'une longue aiguille, du type des aiguilles à ponction lombaire, ou d'une pipette Pasteur, on injecte avec précaution au fond du tube de l'étape d), 5 ml du liquide de densité 1,080 qui présente la composition suivante:

| | |
|---|---|
| Métrizoate de sodium | 15 g (une ampoule de 20 ml à 75 % — produit par. Nyegaard, Oslo) |
| Ficoll (Sigma) | 12,867 g |
| H$_2$O          q.s.p. | 165,8 ml |

on adapte la densité de façon précise à 1,080; si on utilise des ampoules de métrizoate de 30 ml à 32,8 %, la quantité de Ficoll nécessaire pour une ampoule est de 8,441 g avec H$_2$O q.s.p. 108,85 ml.

Cette injection soulève le sang dilué susjacent.

f) On prend la précaution de ne pas agiter pour que les deux milieux ne se mélangent pas, puis l'on procède à une centrifugation de 30 minutes à 400 g à l'interface (par exemple, dans une centrifugeuse Jouan à 1800 tours/minute, avec étoile B).

g) Au terme de la centrifugation, on observe de bas en haut, environ 1 cm d'hématies, puis le liquide de densité 1,080, qui est séparé du tampon, qui se trouve à la partie supérieure, par un anneau qui contient les basophiles et d'autres cellules blanches.

A l'aide d'une seringue munie d'un cathéter, d'une longue aiguille ou à l'aide d'une pipette Pasteur, on prélève avec précaution l'anneau, soit environ 1 à 2 ml de liquide.

On lave cet anneau une fois dans 3 à 5 volumes de tampon Hépès CM; on jette le surnageant pour ne garder qu'environ 0,5 cm$^3$ du sédiment cellulaire dilué dans le tampon.

On remet, sous agitation, les cellules en suspension dans le tampon et on peut ensuite garder la suspension à 4°C, jusqu'à son utilisation dans les deux ou trois heures qui suivent.

La suspension cellulaire obtenue contient de 5 à 10 % de basophiles en suspension dans 0,5 ml de tampon, c'est-à-dire que, alors que le nombre de basophiles contenus dans 1 µl de sang circulant ne dépasse pas 30 ou 40, dans la suspension cellulaire obtenue conformément au procédé objet de l'invention, on obtient un enrichissement de la teneur en basophiles tel que la suspension contient de 300 à 1 000 basophiles par µl.

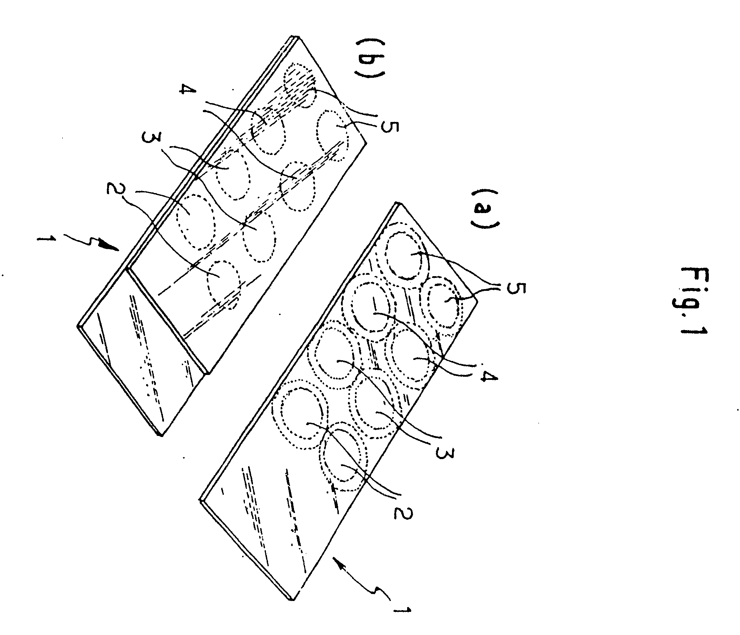

h) Sur des lames de lecture de diagnostic préparées (cf. Figure 1 annexée dans laquelle ces lames sont désignées par la référence 1), c'est-à-dire sur lesquelles est collé un ruban adhésif préalablement percé de trous de diamètre identique, qui constituent des puits 2, 3, 4, 5 (cf. Figure 1), on dépose à l'intérieur de chacun des puits 3, 4, 5, 20 µl de la suspension cellulaire susdite, les cellules en suspension étant réparties de façon régulière dans chaque puit. Dans un certain nombre de puits, le quart d'entre eux par exemple, désignés en référence 2, l'on ajoute à la suspension cellulaire, 10 µl du tampon Hépès CM ci-dessus; dans les autres puits 3, 4, 5, on ajoute à la suspension cellulaire, 10 µl de l'antigène spécifique de la parasitose ou de l'allergie que l'on cherche à diagnostiquer, dilués dans du tampon Hépès CM.

A noter que l'antigène spécifique de la parasitose ou de l'allergie que l'on cherche à diagnostiquer peut avantageusement provenir d'un prélèvement effectué chez un patient atteint de la même parasitose ou allergie, de liquide tumoral ou de liquide allergène qui est conservé, jusqu'à utilisation, par congélation à une température optimale de −80°C.

Dans l'exemple de réalisation présentement décrit, l'antigène spécifique d'une parasitose à diagnostiquer provient de prélèvements du contenu liquide d'un kyste hydatique du foie, lui-même prélevé pendant une intervention chirurgicale, et conservé à −80°C.

Pour utiliser ledit liquide hydatique en tant qu'antigène, on le décongèle, après quoi on peut éventuellement, mais non obligatoirement, le centrifuger à 1000 g pendant 15 minutes.

11

Le surnageant d'une part, le culot de centrifugation d'autre part, peuvent être tous deux utilisés en tant qu'antigène, soit à l'état pur, soit dilués avec un tampon Hépès CM.

i) La lame préparée de la sorte est placée dans une boîte de plastique, dont la teneur en humidité est voisine de la saturation.

On met cette boîte à l'étuve à 37°C durant 15 minutes, temps nécessaire et suffisant pour que la réaction entre la suspension cellulaire et l'antigène puisse avoir lieu.

j) On sort ensuite la boîte de l'étuve, puis on sèche trés rapidement la lame par soufflage d'air chaud. La lame est alors simultanément fixée et colorée à l'aide de la solution suivante:

| | |
|---|---|
| Bleu de toluidine | 2,2 g/litre |
| Sulfate d'alumınium | 7,5 g/litre |
| Chlorure de cétylpyridinium | 0,75 g/litre |
| Glutaraldéhyde | 20 g/litre |
| Formaldéhyde | 35 g/litre |
| Méthanol absolu | 300 ml/litre |
| Ethanol absolu | 300 ml/litre |
| Eau        q.s.p. | 1000 ml |
| pH mesuré | 2,3 |

dans laquelle la lame est trempée pendant 15 minutes, au bout desquelles elle est rincée 30 secondes dans l'eau, 30 secondes dans l'éthanol absolu, et si besoin dans le xylène.

Dans le cas où on utilise des petites boîtes de plastique transparent pour la lecture du diagnostic, on verse directement une quantité appropriée de la solution de colorant fixateur ci-dessus, dans lesdites boîtes pour réaliser la fixation et la coloration rapides, simultanées.

La lame est ensuite montée à l'aide d'un liquide de montage approprié (tel qu' "Eukitt" disponible chez Biolyon) et d'une lamelle (cf. lame b dans la Figure 1).

k) La lecture s'effectue en comptant au microscope (avec un grossissement de 250, 400 ou 1000 en fonction de la richesse en cellules de la suspension) le nombre de basophiles trouvés dans le même nombre de champs microscopiques répartis au hasard dans les puits témoins et dans les puits contenant de l'antigène.

La fixation et la coloration simultanées permettent une identification rapide et parfaite des basophiles intacts dont le nombre (200 à 1000/µl) et l'aspect permettent un compte voisin d'au moins 30 à 50 basophiles par plage-témoin, ce qui est compatible avec une analyse statistique.

La dégranulation des basophiles qui ont réagi avec l'antigène spécifique, s'accompagne d'une disparition apparente de ces cellules qui ne peuvent alors être identifiées que par leur métachromasie, au microscope optique.

La diminution du nombre des basophiles dans les puits contenant l'antigène par rapport aux puits témoins, est exprimée par l'index de dégranulation:

$$ID = \frac{PB\ \text{témoins} - PB\ \text{antigène}}{PB\ \text{témoins}} \times 100$$

En présence d'une parasitose ou d'une allergie, on obtient un ID significatif, au moins supérieur à 35 %, et fréquemment voisin de 100 %.

Exemple 2

Suivant une variante, on utilise des lames déjà préparées avec de l'antigène sec à l'intérieur des puits, un tel antigène ayant été obtenu par lyophilisation du surnageant obtenu après centrifugation de liquide tumoral, comme décrit à l'Exemple 1, h).

Une telle préparation des lames simplifie considérablement la manipulation, puisqu'il suffit de déposer 20 µl de suspension cellulaire dans tous les puits (dont certains, environ le quart, ne contiennent pas d'antigène et serviront de témoins).

Exemple 3

L'antigène présent dans les puits de la lame est introduit à diverses concentrations: par exemple, dans le cas d'une lame à 8 puits dont 2 puits (les puits 2) ne contiennent pas l'antigène et sont donc des puits témoins, on introduit l'antigène, dans les puits 3 à une dilution de $10^{-4}$, dans les puits 4 à une dilution de $10^{-6}$, et dans les puits 5 à une dilution de $10^{-8}$, avec le tampon Hépès CM mentionné dans l'Exemple 1.

Exemple 4

Le comptage oculaire décrit au k) de l'Exemple 1 peut être remplacé par un comptage automatique à l'aide d'une machine connue du commerce, telle que celle qui est connue sous la dénomination commerciale d'"'Optomax''.

L'on peut utiliser, en variante, des solutions de fixation et de coloration rapides, simultanées des lames préparées, de compositions différentes de celle donnée à l'Exemple 1, j) ci-dessus, pour améliorer encore la coloration desdites lames.

II—Exemples de solutions de fixation et de coloration rapides, simultanees

Exemple 5

| | |
|---|---|
| Bleu de toluidine ou colorant métachromatique analogue | 0,2 à 0,8 % |
| Sulfate d'aluminium | 3 à 10 % |
| Chlorure ou bromure de cétylpyridinium | 0,3 à 0,8 % |
| Glutaraldéhyde | 1,5 à 2,5 % |
| Formaldéhyde à 30 % | 300 ml±10 % |
| Méthanol | 300 ml±10 % |
| Ethanol | 300 ml±10 % |
| Eau       q.s.p. | 1000 ml |

Exemple 6

| | |
|---|---|
| Bleu de toluidine ou colorant métachromatique analogue | 0,2 à 0,8 % |
| Sulfate d'aluminium | 3 à 10 % |
| Chlorure ou bromure de cétylpyridinium | 0,3 à 0,8 % |
| Formaldéhyde à 30 % | 300 ml±10 % |
| Méthanol | 300 ml±10 % |
| Ethanol | 300 ml±10 % |
| Eau       q.s.p. | 1000 ml |

Exemple 7

| | |
|---|---|
| Bleu de toluidine ou colorant métachromatique analogue | 0,2 à 0,8 % |
| Sulfate d'aluminium | 3 à 10 % |
| Chlorure ou bromure de cétylpyridinium | 0,3 à 0,8 % |
| Méthanol | 900 ml±10 % |
| Eau       q.s.p. | 1000 ml |

Exemple 8

| | |
|---|---|
| Bleu de toluidine ou colorant métachromatique analogue | 0,5 % |
| Chlorure ou bromure de cétylpyridinium | 0,5 % |
| Sulfate d'aluminium | 5 % |
| Propylèneglycol | 750 ml±10 % |
| Méthanol | 250 ml±10 % |

13

Exemple 9

| | |
|---|---|
| Bleu de toluidine ou colorant métachromatique analogue | 0,5 % |
| Chlorure ou bromure de cétylpyridinium | 0,5 % |
| Sulfate d'aluminium | 5 % |
| Méthanol | 750 ml±10 % |
| Propylèneglycol | 250 ml±10 % |

Exemple 10

| | |
|---|---|
| Bleu de toluidine ou colorant métachromatique analogue | 0,3 % |
| Chlorure ou bromure de cétylpyridinium | 0,5 % |
| Sulfate d'aluminium | 5 % |
| Propylèneglycol | 400 ml±10 % |
| Eau | 200 ml±10 % |
| Méthanol | 400 ml±10 % |

Exemple 11

| | |
|---|---|
| Bleu de toluidine ou colorant métachromatique analogue | 0,5 % |
| Chlorure ou bromure de cétylpyridinium | 0,5 % |
| Sulfate d'aluminium | 5 % |
| Propylèneglycol | 400 ml±10 % |
| Eau | 200 ml±10 % |
| Méthanol | 400 ml±10 % |

Exemple 12

| | |
|---|---|
| Bleu de toluidine ou colorant métachromatique analogue | 0,3 % |
| Chlorure de cétylpyridinium | 0,5 % |
| Sulfate d'aluminium | 5 % |
| Prolylèneglycol | 400 ml±10 % |
| Eau | 400 ml±10 % |
| Méthanol | 200 ml±10 % |

Exemple 13

| | |
|---|---|
| Bleu de toluidine ou colorant métachromatique analogue | 0,5 % |
| Chlorure de cétylpyridinium | 0,5 % |
| Sulfate d'aluminium | 5 % |
| Propylèneglycol | 400 ml±10 % |
| Eau | 400 ml±10 % |
| Méthanol | 200 ml±10 % |

Le tampon Hépès non destructeur à l'égard des cellules basophiles, utilisé pour reprendre la couche leucocytaire séparée par centrifugation, peut présenter des compositions différentes de celle décrite à l'Exemple 1, d) ci-dessus.

III—Exemples de compositions de tampons Hepes

### Exemple 14

| | |
|---|---|
| Hépès 1 Molaire | 10 ml |
| KCl 10 % | 0,932 ml |
| MgCl$_2$ 10 % | 0,510 ml |
| NaĈl 9 ‰ | 871 ml |
| CaCl$_2$ 10% | 0,550 ml |
| Eau q.s.p. | 1000 ml |
| pH | 7,4—7,6 |

L'osmolarité du tampon est contrôlée de manière à être sensiblement voisine (au moins à 20 % près) de celle du plasma.

### Exemple 15

| | |
|---|---|
| Hépès 1 M | 5 ml |
| KCl 10 % | 0,932 ml |
| MgCl$_2$ 10 % | 0,510 ml |
| NaCl 9 ‰ | 880 ml |
| CaCl$_2$ 510 % | 0,550 ml |
| Eau q.s.p. | 1000 ml |
| pH | 7,4—7,6 |

L'osmolarité du tampon est contrôlée de manière à être sensiblement voisine (au moins à 20 % près) de celle du plasma.

### Exemple 16

| | |
|---|---|
| Tampon Hépès, solution molaire | 4 à 25 ml |
| KCl 10 % | 0,932 ml |
| CaCl$_2$ 10 % | 0,550 ml |
| MgCl$_2$ 10 % | 0,510 ml |
| NaCl 9 ‰ | 850 à 900 ml |
| Héparine | 10 unités/ml |
| Eau q.s.p. | 1000 ml |
| pH | 7,4—7,6 |

L'osmolarité du tampon est contrôlée de manière à être sensiblement voisone (au moins à 20 % près) de celle du plasma.

Exemple 17

| Tampon Hépès, solution molaire | 4 à 25 ml |
|---|---|
| KCl 10 % | 0,932 ml |
| NaCl 9 ‰ | 850 à 900 ml |
| Eau     q.s.p. | 1000 ml |
| pH | 7,4—7,6 |

L'osmolarité du tampon est contrôlée de manière à être sensiblement voisine (au moins à 20 % près) de celle du plasma.

IV—Autre exemple de preparation d'un reactif de diagnostic de parasitoses et d'allergies conforme a la presente invention Exemple 18

1) On reconstitue en milieu tampon Hépès tel que décrit à l'Exemple 15 ci-dessus, c'est-à-dire qu'on prélève 2 ml de milieu tampon concentré dix fois contenu dans une ampoule, auxquels on ajoute 18 ml d'eau distillée. L'on obtient ainsi 20 ml de tampon dit reconstitué dont on prélève 5 ml, qu'on distribue dans un tube centrifugeable de 20 ml. On ajoute à ces 5 ml de tampon Hépès reconstitué 50 à 250 unités internationales d'héparinate de calcium, de lithium ou de sodium. Le tube ainsi préparé est le tube (1).

2) On vide le contenu d'une ampoule de milieu de séparation de basophiles (MSB) prêt à l'emploi, qui est le liquide de densité 1,079—1,085 mentionné dans la présente invention, dans un tube de 20 ml à fond rond centrifugeable. Le tube ainsi préparé est le tube (2).

3) On prélève à jeûn 5 ml de sang veineux sur de l'EDTA K$_3$ ["Vacutainer" (Marque déposée), "Venoject" (Marque déposée) ou à l'aide d'une aiguille à plateau reliée à un tube contenant une quantité d'EDTA K$_3$ telle que la concentration finale d'EDTA K$_3$ dans le sang sera de 1,5 mg/ml de sang].

4) On transfère ce sang dans le tube (1) qui contient le tampon Hépès hépariné (voir 1) ci-dessus) et l'on homogénéise doucement.

5) On dépose doucement dans le tube (2) (voir 2) ci-dessus), à l'aide d'une pipette Pasteur, le mélange de sang et de tampon Hépès homogénéise à la surface du MSB.

6) On centrifuge ce tube pendant 30 minutes à 400 g à l'interface. On obtient à la limite entre le MSB et le milieu tampon, un anneau blanchâtre que l'on prélève en totalité (1 à 3 ml) à l'aide d'une pipette Pasteur montée sur seringue ou poire.

7) Le milieu cellulaire ainsi obtenu, est déposé dans un tube à centrifuger contenant le reste du milieu tampon Hépès hépariné et est lavé par une centrifugation de 10 minutes à 150 g. Le surnageant est prélevé jusqu'à la rupture de pente du tube (volume restant±0,5 ml).

8) Après une homogénéisation douce d'une durée de 30—50 secondes par agitation à la main ou au vortex, le culot cellulaire prêt à l'emploi est prêt pour l'étape suivante de dépôt sur les lames de lecture du diagnostic.

A noter que toutes les opérations ci-dessus, et notamment la centrifugation, sont réalisées à la température ambiante, 20°C environ.

9) Réaction de dégranulation

Dans chacun des puits d'une lame de lecture de diagnostic, on dépose 20 µl de suspension cellulaire (culot resuspendu).

La lame est ensuite mise dans une boîte couverte (type boîte de Pétri) contenant un morceau de coton humide. L'ensemble est placé à l'étuve à 37°C pendant 20 minutes.

Puis le couvercle de la boîte et le coton sont enlevés. La lame, toujours à l'étuve, est ainsi séchée en environ 15 minutes. On peut aussi sécher les lames au sèche-cheveux.

La fixation/coloration est obtenu en recouvrant chacun des puits d'une solution de coloration et de fixation rapides, simultanées conforme à l'un des Exemples 5 à 13 ci-dessus. La lame est laissée sur la paillasse pendant 15 minutes, puis est rincée sous un filet d'eau du robinet (30 secondes), déshydratée dans un bain d'éthanol absolu (30 secondes), et plongée une seconde dans un bain de xylène, pour faciliter le montage.

Une goutte de milieu de montage est déposée sur une lamelle. Celle-ci est ensuite appliquée sur la lame. 10 à 15 minutes sont nécessaires pour sécher l'ensemble (on peut accélérer le séchage en mettant la lame à l'étuve ou sous un sèche-cheveux).

10) Lecture du test

Elle est réalisée au microscope optique ordinaire. Il est préférable de disposer d'objectifs et d'oculaires "grand-champ" et de qualité suffisante pour procurer une mise au point parfaite sur l'ensemble du champ.

Le grandissement utilisé est variable avec le nombre de basophiles. En général de 400, il peut être porté à 1000 (immersion) en cas de grande richesse en basophiles, ou au contraire à 250 si l'observateur est habitué.

Les basophiles sont reconnus grâce à leur coloration foncée par rapport aux autres cellules

16

(essentiellement lymphocytes et monocytes), et surtout à la teinte violette parfois rouge différente de la coloration bleue (claire ou foncée) des autres cellules.

Le basophiles sont comptés sur chacun des puits. Dans la grande majorité des cas, vingt champs microscopiques sont suffisants (dix champs au hasard dans un diamètre vertical et dix champs au hasard dans un diamètre horizontal), L'important est, bient entendu, qu'un même nombre de champs soit examiné sur chacun des puits.

En ajoutant les nombres de basophiles trouvés dans les puits identiques A et B, on obtient:

T=nombre de basophiles sans antigène

Ag (1, 2, 3 ou 4)=nombre de basophiles avec antigène.

On détermine l'index de dégranulation (I.D.) pour chaque dilution d'antigène par la relation suivante:

$$\text{I.D. } \% = \frac{T - Ag}{T} \times 100$$

V—Exemple d'application du procede de diagnostic conforme a l'invention

L'antigène utilisé a été obtenu à partir du contenu liquide d'un kyste hydatique du foie, prélevé pendant l'intervention chirurgicale et conservé à −80°C.

Après décongélation, le liquide hydatique a été centrifugé à 1000 g pendant 15 minutes. Le surnageant d'une part, le culot de centrifugation d'autre part, ont été employés comme antigènes purs ou dilués avec un tampon Hépès CM.

Le résultats ont été exprimés en pourcentage de basophiles ayant apparemment disparu en présence d'antigène par rapport au nombre de basophiles comptés en présence du tampon sans antigène.

L'observation concerne un homme âgé de 38 ans, qui avait un kyste hydatique de 15 cm de diamètre, développé aux dépens du lobe droit du foie. Son contenu était translucide avec d'innombrables vésicules filles. Le kyste et l'adventice hydatique ont fait l'objet d'une exérèse complète. L'immunodiagnostic (électrophorèse et hémagglutination) était, avant l'intervention, négatif; le quatrième jour post-opératoire, l'hémagglutination était positive à 1/12800 et l'injection intradermique de dilutions décroissantes du liquide stérilisé par filtration, a montré une réaction positive à 1/100 (15—35), à la 20 ème minute.

Le test conforme à l'invention a été fait, chez le malade, le deuxième et le quatrième jours post-opératoires et chaque fois chez deux témoins. Les quatre témoins étaient indemnes de toute infestation parasitaire. L'un des témoins avait un hémolymphangiome de 3420 grammes du lobe droit du foie qui a été réséqué.

Resultats

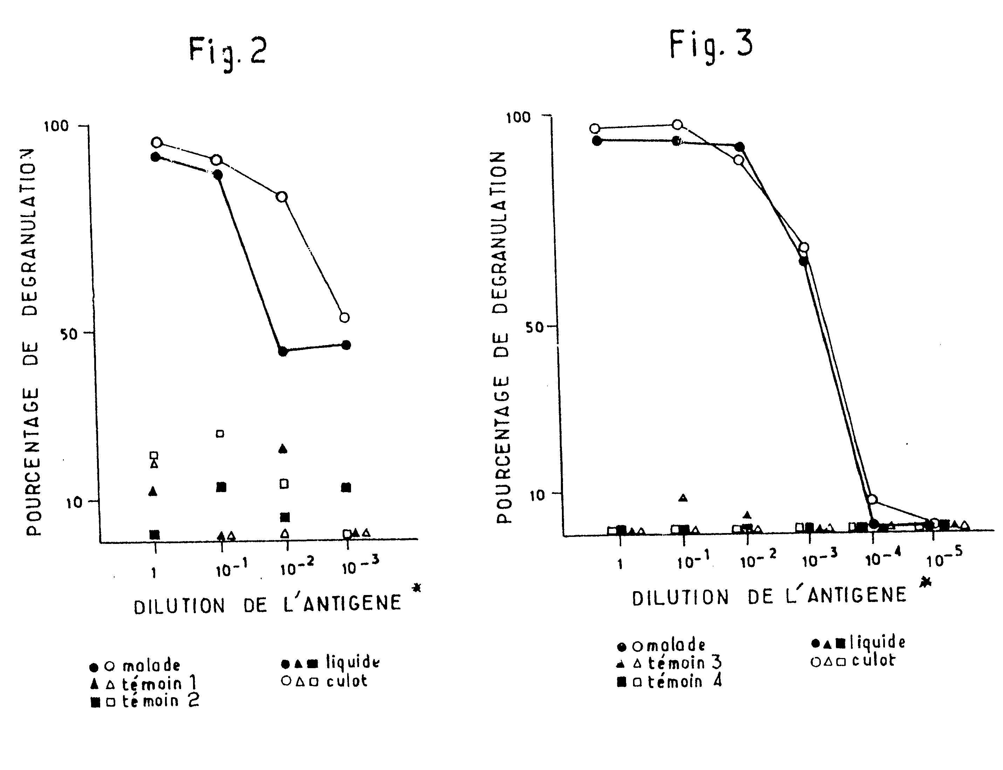

Les Figures 2 et 3 montrent les pourcentages de dégranulation du malade et des deux témoins au deuxième jour et au quatrième jour post-opératoires. Ces pourcentages ont été établis sur plus de 200 basophiles (nombre réellement compté et non extrapolé à partir de quelques cellules dénombrées sur lame de Mallasez) pour chaque sujet (de 202 à 1270) en l'absence d'antigène.

Ces résultats montrent, chez ce malade atteint d'un kyste hydatique du foie, une dégranulation des basophiles en présence de liquide hydatique, très hautement significative.

Il n'a pas été observé de différences dans les résultats selon que l'antigène utilisé était le liquide hydatique ou le culot de centrifugation.

Il résulte de la description qui précède que l'on obtient, conformément à l'invention, des moyens pour la réalisation d'un test de diagnostic de parasitoses et d'allergies comportant une élévation des IgE spécifiques, qui est extrèmement fiable et donne un pourcentage de concordance par rapport aux tests de l'histamine, qui est voisin de 100 %.

De plus, la lecture des lames de diagnostic est considérablement facilitée et accélérée par l'augmentation du nombre des basophiles à compter, permettant ainsi d'obtenir des index de dégranulation statistiquement valables.

. Le réactif de diagnostic et le test de diagnostic conformes à la présente invention, sont applicables ou diagnostic de toutes les parasitoses et allergies qui déterminent une augmentation des IgE spécifiques, et notamment aux helminthiases telles que bilharziose, ténia, ascariose, filariose, oxyurose, etc.

**Revendications**

1. Procédé de diagnostic des parasitoses et des allergies, et en particulier des parasitoses à vers et des allergies qui provoquent une augmentation des immunoglobulines spécifiques circulantes, et notamment des IgE, qui consiste:— à mettre en contact pendant environ 15 minutes à 37°C, un réactif de diagnostic des parasitoses et des allergies constitué par une suspension de polynucléaires basophiles porteurs d'IgE spécifiques, dans un tampon approprié, avec de l'antigène spécifique de la parasitose ou de l'allergie que l'on cherche à diagnostiquer, contenu à différentes concentrations dans un certain nombre de puits d'une lame ou d'une boîte de lecture de diagnostic, tandis que les puits restants de ladite lame ou boîte contiennent le réactif de diagnostic seul, pour servir de témoins; — à procéder à un séchage rapide de la lame ou boîte ainsi préparée; — à procéder à la fixation et à la coloration rapides de la lame préparée de la sorte, à l'aide de moyens de fixation et de coloration rapides qui colorent sélectivement les granules cytoplasmiques des basophiles; — à compter les polynucléaires basophiles (PB) au microscope optique, avec un grossissement approprié, respectivement dans les puits témoins et dans les différents puits contenant le réactif et différentes concentrations d'antigène; — et à établir, par comparaison de la diminution du nombre de polynucléaires basophiles dans les puits contenant l'antigène à diverses concentrations, par rapport au nombre de polynucléaires basophiles contenus dans les puits témoins, l'index de dégranulation:

$$ID = \frac{PB\ témoins - PB\ antigène}{PB\ témoins} \times 100$$

ou pourcentage de basophiles qui ont apparemment disparu en présence de l'antigène, qui, s'il est supérieur à 35 %, permet de diagnostiquer la présence de la parasitose ou de l'allergie recherchée, lequel procédé est caractérisé en ce que le réactif de diagnostic introduit dans les puits de la lame ou de la boîte susdite, est constitué par une suspension enrichie en polynucléaires basophiles, dans un tampon non destructeur vis-à-vis des cellules basophiles, tel que le tampon Hépès CM ou le tampon Pipès, notamment, laquelle suspension contient de 300 à 1000 basophiles par µl et a été obtenu à partir d'un échantillon de sang prélevé chez un patient humain ou animal supposé atteint de la parasitose ou de l'allergie à diagnostiquer, lequel échantillon de sang a été soumis à l'un ou l'autre des traitements suivants:

— ou bien l'échantillon de sang est soumis à une centrifugation, pendant une durée et à une vitesse appropriées, pour recueillir une couche leucocytaire et un plasma surnageant riche en plaquettes, lequel a été rejeté, tandis que la couche leucocytaire recueillie a été mélangée avec une quantité appropriée d'un tampon non destructeur à l'égard des cellules basophiles tel que le tampon Hépès CM ou le tampon Pipès, notamment;
— ou bien ledit échantillon de sang total est mélangé avec un sel de l'acide éthylènediaminetétraacétique, puis mélangé avec un tampon Hépès de composition appropriée;
— ou bien ledit échantillon de sang total est mélangé avec l'acide éthylènediaminetétraacétique avantageusement sous la forme de l'un de ses sels, et de préférence sous la forme de son sel potassium (EDTA K$_3$), puis mélangé avec un tampon Hépès de composition appropriée contenant de l'héparine à 5—20 unités/ml;
— après quoi, un liquide de densité 1,079—1,085 dont l'osmolarité est comprise entre 280 et 320 milliosmoles, avantageusement constitué par une composition comprenant du métrizoate de sodium et du Ficoll ou du Percol, est injecté sous le mélange de couche leucocytaire et de tampon ou sous le mélange de sang total prélevé sur sel d'acide d'éthylènediaminetétraacétique, avec un tampon éventuellement hépariné,
— le mélange obtenu étant centrifugé avec ledit liquide de densité pour obtenir un anneau contenant une concentration élevée en basophiles,
— lequel anneau est prélevé, puis remis en suspension dans un tampon tel que le tampon susdit;
— ledit procédé étant, en outre, caractérisé en ce que les moyens de fixation et de coloration rapides susdits sont constitués par une composition unique qui réalise simultanément la fixation et la coloration rapides et qui comprend un colorant métachromatique, qui est de préférence du bleu de toluidine, associé à du sulfate d'aluminium, à un halogénure de cétylpyridinium et:— — —
— soit à du glutaraldéhyde et à du formaldéhyde, en solution dans un mélange d'éthanol et de méthanol absolus et d'eau, laquelle solution a un pH acide inférieur à 2,5;
— soit à du méthanol éventuellement associé à du formaldéhyde et/ou à de l'éthanol, en l'absence de glutaraldéhyde;
— soit à du propylèneglycol associé à du méthanol, en l'absence de glutaraldéhyde, de formaldéhyde et d'éthanol.

2. En tant que réactif de fixation et de coloration rapides, simultanées, nécessaire à la mise en oeuvre du procédé de diagnostic selon la Revendication 1, une solution hydroalcoolique à pH acide, inférieur à 2,5 caractérisée en ce qu'elle présente la composition suivante:

**0 022 698**

| | |
|---|---|
| Bleu de toluidine ou colorant métachromatique analogue | 2,2 g/litre |
| Sulfate d'aluminium | 7,5 g/litre |
| Halogénure de cétylpyridinium | 0,75 g/litre |
| Glutaraldéhyde | 20 g/litre |
| Formaldéhyde | 35 g/litre |
| Méthanol absolu | 300 ml/litre |
| Ethanol absolu | 300 ml/litre |
| Eau          q.s.p. | 1000 ml |

3. Réactif de fixation et de coloration rapides, simultanées, nécessaire à la mise en oeuvre du procédé de diagnostic selon la Revendication 1, caractérisé en ce qu'il présente la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Glutaraldéhyde | 1,5 à 2,5 % |
| Formaldéhyde à 30 % | 270 à 330 ml |
| Méthanol | 270 à 330 ml |
| Ethanol | 270 à 330 ml |
| Eau          q.s.p. | 1000 ml |

4. Réactif de fixation et de coloration rapides, simultanées, nécessaire à la mise en oeuvre du procédé de diagnostic selon la Revendication 1, caractérisé en ce qu'il présente la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Méthanol | 300 ml±10 % |
| Formaldéhyde à 30 % | 300 ml±10 % |
| Ethanol | 300 ml±10 % |
| Eau          q.s.p. | 1000 ml |

5. Réactif selon la Revendication 1, caractérisé en ce qu'il présente la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Méthanol | 830 à 990 ml |
| Eau          q.s.p. | 1000 ml |

19

**0 022 698**

6. Réactif de fixation et de coloration rapides, simultanées, nécessaire à la mise en oeuvre du procédé de diagnostic selon la Revendication 1, caractérisé en ce qu'il présente la composition suivante:

| | |
|---|---|
| Bleu de toluidine ou colorant métachromatique analogue | 0,2 à 0,8 % |
| Sulfate d'aluminium | 3 à 10 % |
| Halogénure de cétylpyridinium | 0,3 à 0,8 % |
| Propylèneglycol | 250 ml à 600 ml±10 % |
| Méthanol | 200 ml à 750 ml±10 % |
| Eau          q.s.p. | 1000 ml |

7. Réactif selon l'une quelconque des Revendications 2 à 6, caractérisé en ce que l'halogénure de cétylpyridinium mis en oeuvre, est le chlorure ou le bromure de cétylpyridinium.

8. Ensemble prêt à l'emploi, ou kit, de préférence contenu dans un coffret unique, pour la réalisation du test de diagnostic des parasitoses et des allergies, selon la Revendication 1, caractérisé en ce qu'il comprend, en combinaison:— une pluralité de lames ou boîtes présentant chacune un certain nombre de puits de diamètre égal dont la majeure partie, à l'exception des puits devant constituer les témoins, contiennent une quantité prédéterminée d'un antigène spécifique d'une parasitose ou d'une allergie à diagnostiquer, lequel antigène est introduit à l'état sec, de préférence lyophilisé, dans chacun des puits, lesdites quantités prédéterminées variant d'une colonne de puits à la suivante et lesdits puits étant destinés à recevoir, pour la réalisation du test de diagnostic selon la Revendication 1, une quantité appropriée d'un réactif de diagnostic d'une parasitose ou d'une allergie; — une pluralité de tubes contenant des quantités prédosées d'un tampon approprié tel que le tempon Hépès CM ou Pipès; — une pluralité de récipients adaptés tels que des ampoules, des tubes ou des seringues contenant chacun un liquide de densité 1,079—1,085; — au moins un récipient contenant une solution hydroalcoolique de fixation et de coloration rapides, simultanées, des lames, boîtes ou analogues susdites, conforme à l'une quelconque des Revendications 1 à 7.

9. Ensemble prêt à l'emploi, ou kit, selon la Revendication 8, caractérisé en ce que:— chacun des puits contenant un antigène spécifique de la parasitose ou de l'allergie à diagnostiquer contient 10 à 20 µl dudit antigène à l'état lyophilisé et est propre à recevoir, lors de la réalisation du test de diagnostic selon la Revendication 1, des quantités variables croissantes, comprises entre 3 et 20 µl de réactif de diagnostic; — chaque tube contenant une quantité prédosée de tampon, contient 5 ml dudit tampon; — chaque récipient adapté tel que tube, ampoule ou seringue contenant du liquide de densité contient 5 ml dudit liquide de densité 1,079—1,085; — le ou chaque récipient contenant la solution hydroalcoolique de fixation et de coloration rapides, simultanées, des lames ou boîtes préparées, renferme une solution à pH inférieur à 2,5, présentant la composition selon la Revendication 2.

10. Kit ou ensemble prêt à l'emploi pour la réalisation du test de diagnostic des parasitoses et des allergies selon la Revendication 1, caractérisé en ce que le tampon Hépès contenu en quantités prédosées dans des ampoules ou des tubes appropriés, comprend de 4 à 25 ml d'une solution molaire de tampon Hépès, 0,932 ml de KCl à 10 %, 0,550 ml de CaCl$_2$ à 10 %, 0,510 ml de MgCl$_2$ à 10 %, de 850 à 900 ml de NaCl à 9 ‰, en solution dans une quantité d'eau suffisante pour la porter à 1000 ml, ou/et en ce que la solution de fixation et de coloration rapides, simultanées, des préparations antigéniques (ou témoins) portées par les lames de lecture du diagnostic, contenu en quantités prédosées dans des ampoules ou des tubes unitaires appropriés, présente la composition selon l'une quelconque des Revendications 2 à 7.

11. Kit ou ensemble prêt à l'emploi selon la Revendication 10, caractérisé en ce qu'il se compose d'un coffret qui comprend:— une pluralité de récipients adaptés tels que des ampoules ou des tubes contenant des quantités prédosées d'un tampon Hépès approprié, et notamment d'un tampon Hépès comprenant 4 à 0,25 ml d'une solution molaire de tampon Hépès, 0,932 ml de KCl à 10 %, 0,550 ml de CaCl à 10 %, 0,510 ml de MgCl$_2$ à 10 %, 850 à 900 ml de NaCl à 9 ‰, dans une quantité d'eau suffisante pour la portée à 1000 ml, cette solution ayant un pH de 7,4—7,6, — une pluralité d'ampoules, de tubes ou de seringues contenant des quantités prédosées d'un liquide de densité 1,079—1,085, — une pluralité de lames ou de boîtes de lecture de diagnostic, présentant chacune un certain nombre de puits, dont la majeure partie, à l'exception des puits témoins, sont destinés à recevoir des quantités prédéterminées variables d'un antigène spécifique d'une allergie ou d'une parasitose à diagnostiquer, ainsi que des quantités prédéterminées variables de réactif de diagnostic, — et une pluralité d'ampoules ou de tubes renfermant des quantités prédosées d'une solution de fixation et de coloration rapides, simultanées, selon l'une quelconque des Revendications 2 à 7.

12. Kit ou ensemble prêt à l'emploi selon la Revendication 10, caractérisé en ce qu'il se compose de deux coffrets séparés dont l'un, qui est destiné à la séparation des cellules basophiles, comprend:— une pluralité de récipients adaptés, tels que des ampoules ou des tubes contenant des quantités prédosées d'un tampon approprié, et notamment de tampon Hépès selon la Revendication 11, — une pluralité d'ampoules ou de tubes contenant chacun des quantités prédosées de liquide de densité 1,079—1,085; et

20

dont l'autre coffret comprend:— une pluralité de lames ou de boîtes de lecture de diagnostic, présentant chacune un certain nombre de puits dont la majeure partie, à l'exception des puits témoins, sont destinés à recevoir des quantités prédéterminées variables d'un antigène spécifique d'une allergie ou d'une parasitose à diagnostiquer, ainsi que des quantités prédéterminées variables de réactif de diagnostic, — et une pluralité d'ampoules ou de tubes renfermant des quantités prédosées d'une solution de fixation et de coloration rapides, simultanées, du type des solutions selon l'une quelconque des Revendications 2 à 7, pour la réalisation du test de diagnostic selon la Revendication 1.

13. Kit ou ensemble prêt à l'emploi selon la Revendication 10, en un ou en deux coffrets, caractérisé en ce que le tampon Hépès contenu en quantités prédosées dans une pluralité de récipients adaptés tels que tubes ou ampoules, est constitué par une solution aqueuse comprenant 4 à 25 ml d'une solution molaire de tampon Hépès, 0,932 ml de KCl à 10 %, 0,550 ml de $CaCl_2$ à 10 %, 0,510 ml de $MgCl_2$ à 10 %, 850 à 900 ml de NaCl à 9 ‰, 10 à 15 unités/ml d'héparine lesquels constituants sont dissous dans une quantité d'eau suffisante pour porter la solution à 1000 ml, cette solution présentant un pH compris entre 7,4 et 7,6.

14. Kit ou ensemble prêt à l'emploi selon la Revendication 10, en un ou en deux coffrets, caractérisé en ce que le tampon Hépès contenu en quantités prédosées dans une pluralité de récipients adaptés tels que tubes ou ampoules, est constitué par une solution aqueuse comprenant 4 à 25 ml d'une solution molaire de tampon Hépès, 0,932 à 1,2 ml de KCl à 10 %, 850 à 900 ml de NaCl à 9 ‰, lesquels constituants sont dissous dans une quantité d'eau suffisante pour porter la solution à 1000 ml, cette solution présentant un pH compris entre 7,4 et 7,6, et en ce que les lames ou les boîtes de lecture de diagnostic comportent, dans chacun de leurs puits destinés à recevoir de l'antigène, une quantité prédéterminée de calcium et de magnésium sous la forme de leurs sels, et notamment de leurs chlorures.

15. Kit ou ensemble prêt à l'emploi selon la Revendication 14, caractérisé en ce que le calcium est présent sur les lames ou les boîtes de lecture du diagnostic à raison d'environ 1 mmole/litre, et le magnésium est présent à raison d'environ 1 mmole/litre.

16. Réactif de diagnostic pour la réalisation du test de diagnostic selon la Revendication 1, en particulier à l'aide du Kit selon l'une quelconque des Revendications 9 à 15, caractérisé en ce que ledit réactif est constitué par une suspension enrichie en polynucléaires basophiles porteurs d'IgE spécifiques, qui contient de 300 à 1000 basophiles par µl et que a été obtenue à partir d'un échantillon de sang prélevé chez un patient humain ou animal supposé atteint de la parasitose ou de l'allergie à diagnostiquer, lequel échantillon de sang a été soumis à l'un ou l'autre des traitements suivants:

— ou bien l'échantillon de sang est soumis à une centrifugation, pendant une durée et à une vitesse appropriées, pour recueillir une couche leucocytaire et un plasma surnageant riche en plaquettes, lequel a été rejeté, tandis que la couche leucocytaire recueillie a été mélangée avec une quantité appropriée d'un tampon non destructeur à l'égard des cellules basophiles tel que le tampon Hépès CM ou le tampon Pipès, notamment;

— ou bien ledit échantillon de sang total est mélangé avec un sel de l'acide éthylènediaminetétraacétique, puis mélangé avec un tampon Hépès de composition appropriée;

— ou bien ledit échantillon de sang total est mélangé evec l'acide éthylènediaminetétraacétique avantageusement sous la forme de l'un de ses sels, et de préférence sous la forme de son sel de potassium (EDTA $K_3$), puis mélangé avec un tampon Hépès de composition appropriée contenant de l'héparine à 5—20 unités/ml;

— après quoi, un liquide de densité 1,079—1,085 dont l'osmolarité est comprise entre 280 et 320 milliosmoles, avantageusement constitué par une composition comprenant du métrizoate de sodium et du Ficoll ou du Percol, est injecté sous le mélange de couche leucocytaire et de tampon ou sous le mélange de sang total prélevé sur sel d'acide d'éthylènediaminetétraacétique, avec un tampon éventuellement hépariné,

— le mélange obtenu étant centrifugé avec ledit liquide de densité pour obtenir un anneau contenant une concentration élevée en basophiles,

— lequel anneau est prélevé, puis remis en suspension dans un tempon tel que le tampon susdit,

— la suspension ainsi formée étant prête à être déposée dans les puits d'une lame ou d'une boîte de lecture de diagnostic.

17. Réactif selon la Revendication 16, caractérisé en ce que le tampon non destructeur à l'égard des cellules basophiles auquel est mélangée la couche leucocytaire obtenue après centrifugation, et dans lequel est mis en suspension l'anneau riche en basophiles obtenu après centrifugation du mélange ci-dessus sous lequel a été injecté ledit liquide de densité est un tampon qui présente la composition suivante:

| | |
|---|---|
| Tampon Hépès, solution molaire | 25 ml |
| KCl 10 % | 0,932 ml |
| CaCl$_2$ 10 % | 0,550 ml |
| MgCl$_2$ 10 % | 0,510 ml |
| NaCl 9 ‰ (7,6 g) | 844,11 ml |
| H$_2$O        q.s.p. | 1000 ml |

18. Réactif selon la Revendication 17, caractérisé en ce que le tampon non destructeur à l'égard des cellules basophiles auquel est mélangée la couche leucocytaire obtenue après centrifugation, et dans lequel est mis en suspension l'anneau riche en basophiles obtenu après centrifugation du mélange ci-dessus sous lequel a été injecté ledit liquide de densité est un tampon qui présente la composition suivante:

| | |
|---|---|
| Tampon Hépès, solution molaire | 4 à 25 ml |
| KCl 10 % | 0,932 ml |
| CaCl$_2$ 10 % | 0,550 ml |
| MgCl$_2$ 10 % | 0,510 ml |
| NaCl 9 ‰ | 850 à 900 ml |
| Eau        q.s.p. | 1000 ml |
| pH | 7,4—7,6 |

19. Réactif de diagnostic selon la Revendication 16, caractérisé en ce que le tampon auquel est mélangé l'échantillon de sang total prélevé sur un sel d'éthylènediaminetétraacétique et éventuellement le tampon dans lequel l'anneau riche en basophiles résultant de la centrifugation est mis en suspension, présente la composition suivante:

| | |
|---|---|
| Tampon Hépès, solution molaire | 4 à 25 ml |
| KCl 10 % | 0,932 ml |
| CaCl$_2$ 10 % | 0,550 ml |
| MgCl$_2$ 10 % | 0,510 ml |
| NaCl 9 % | 850 à 900 ml |
| Héparine | 10 à 15 unités/ml |
| Eau        q.s.p. | 1000 ml |
| pH | 7,4—7,6 |

20. Réactif de diagnostic selon la Revendication 16, caractérisé en ce que le tampon dans lequel est mélangé l'échantillon de sang total prélevé sur un sel d'éthylènediaminetétraacétique et éventuellement le tampon dans lequel l'anneau riche en basophiles résultant de la centrifugation est mis en suspension, présente la composition suivante:

| | |
|---|---|
| Tampon Hépès, solution molaire | 4 à 25 ml |
| KCl 10 % | 0,932 à 1,2 ml |
| NaCl 9 ‰ | 850 à 900 ml |
| Eau        q.s.p. | 1000 ml |
| pH | 7,4—7,6 |

**0 022 698**

à condition que le calcium et le magnésium nécessaires soient déposés sous la forme de leurs sels, en quantités prédéterminées sur les lames ou les boîtes de lecture du diagnostic. conformément au procédé objet de la Revendication 1.

**Patentansprüche**

1. Verfahren zur Diagnose von Parasitosen und Allergien, insbesondere von Wurm-Parasitosen und Allergien, die eine Vermehrung der spezifischen zirkulierenden Immunglobuline und insbesondere der IgE hervorrufen, das darin besteht: während etwa 15 Minuten bei 37°C ein diagnostisches Reagens für Parasitosen und Allergien, das aus einer Suspension polynuklearer basophiler Träger spezifischer IgE in einem geeigneten Puffer besteht, mit dem spezifischen Antigen der Parasitose oder der Allergie, die diagnostisch festgestellt werden soll, enthalten in verschiedenen Konzentrationen in einer bestimmten Anzahl von Vertiefungen einer Platte oder eines diagnostischen Ablesekastens enthalten ist, in Kontakt zu bringen, wobei die restlichen Vertiefungen der Platte oder des Kastens nur diagnostisches Reagens enthalten, um als Kontrollen zu dienen;

— eine rasche Trocknung der so bereiteten Platte oder des so bereiteten Kastens durchzuführen;
— eine rasche Fixierung und Färbung der derart präparierten Platte mit raschen Fixier- und Färbemitteln, die die Cytoplasmagranulate der Basophile selektiv färben, durchzuführen;
— die polynuklearen Basophile (PB) im optischen Mikroskop mit einer geeigneten Vergrößerung jeweils in den Kontrollvertiefungen und den verschiedenen Vertiefungen, die das Reagens und die verschiedenen Antigenkonzentrationen enthalten, zu zählen;
— und durch Vergleich der Verringerung der Anzahl der polynuklearen Basophile in den Vertiefungen, die das Antigen in verschiedenen Konzentrationen enthalten, mit der Anzahl der polynuklearen Basophile, die in den Kontrollvertiefungen enthalten sind, den Degranulationsindex:

$$ID = \frac{PB \text{ Kontrollen} - PB \text{ Antigen}}{PB \text{ Kontrollen}} \times 100$$

oder den Prozentsatz der Basophile, die offensichtlich in Anwesenheit des Antigens verschwunden sind, zu bestimmen, der, wenn er über 35 % liegt, die Diagnose der Anwesenheit der gesuchten Parasitose oder Allergie ermöglicht, wobei dieses Verfahren dadurch gekennzeichnet ist, daß das in die Vertiefungen der Platte oder des genannten Kastens eingeführte diagnostische Reagens aus diner Suspension besteht, die mit polynuklearen Basophilen angereichert ist, in einem gegenüber den basophilen Zellen nicht zerstörend wirkenden Puffer, wie insbesondere Hepes CM-Puffer oder Pipes Puffer, wobei die Suspension 300 bis 1000 Basophile pro µl enthält, und erhalten wurde ausgehend von einer Blutprobe, die einem menschlichen oder tierischen Patienten entnommen wurde, von dem angenommen wird, daß er von der zu diagnostizierenden Parasitose oder Allergie befallen ist, wobei diese Blutprobe der einen oder anderen der folgenden Behandlungen unterzogen wurde:
— entweder wird die Blutprobe einer Zentrifugierung während einer geeigneten Dauer und Geschwindigkeit unterworfen, um eine Leukozytenschicht und ein überstehendes mit Plättchen angereichertes Plasma zu erzielen, welches verworfen wurde, während die gewonnene Leukozytenschicht mit einer geeigneten Menge eines Puffers vermischt wurde, der auf die basophilen Zellen nicht zerstörend wirkt, wie insbesondere Hepes CM-Puffer oder Pipes-Puffer;
— oder wird die gesamte Blutprobe mit einem Salz der Ethylendiamintetraessigsäure vermischt und anschliessend mit einem Hepes Puffer geeigneter Zusammensetzung vermischt;
— oder wird die gesamte Blutprobe mit Ethylendiamintetraessigsäure, vorteilhaft in der Form eines ihrer Salze und vorzugsweise in der Form ihres Kaliumsalzes (EDTA $K_3$) vermischt und anschließend mit einem Hepes Puffer geeigneter Zusammensetzung, der Heparin zu 5—20 Einheiten/ml enthält, vermischt;
— worauf eine Flüssigkeit mit einer Dichte von 1,079—1,085, deren Osmolarität bei 280 bis 320 Milliosmol liegt, die vorteilhaft aus einer Zusammensetzung besteht, die Natriummetrizoat und Ficoll oder Percol enthält, unter das Gemisch der Leukozytenschicht und des Puffers oder unter das Gemisch des gesamten über dem Salz der Ethylendiamintetraessigsäure entnommenen Blutes mit einem gegebenenfalls heparinisierten Puffer, injiziert,
— wobei das erhaltene Gemisch mit der Flüssigkeit mit der Dichte zentrifugiert wird, um einen Ring zu erhalten, der eine erhöhte Konzentration an Basophilen enthält,
— dieser Ring wird entnommen und anschließend erneut in einem Puffer, wie dem vorstehend genannten Puffer, suspendiert;
— wobei dieses Verfahren außerdem dadurch gekennzeichnet ist, daß die vorstehend genannten raschen Fixier- und Färbemittel aus einer einzigen Zusammensetzung bestehen, die gleichzeitig die rasche Fixierung und Färbung bewirkt, und die ein metachromatisches Färbemittel enthält, das vorzugsweise Toluidinblau ist, assoziiert mit Aluminiumsulfat, einem Cetylpyridiniumhalogenid und — entweder

23

Glutaraldehyd und Formaldehyd, in Lösung in einem Gemisch von absolutem Ethanol und absolutem Methanol und Wasser, wobei die Lösung einen sauren pH-Wert unter 2,5 hat;
— oder Methanol, gegebenenfalls assoziiert mit Formaldehyd und/oder Ethanol in Abwesenheit von Glutaraldehyd;
— oder Propylenglykol, assoziiert mit Methanol, in Abwesenheit von Glutaraldehyd, Formaldehyd und Ethanol.

2. Als Reagens zur gleichzeitigen raschen Fixierung und Färbung, notwendig zur Durchführung des diagnostischen Verfahrens nach Anspruch 1, eine wässrig-alkoholische Lösung mit saurem pH-Wert unter 2,5, dadurch gekennzeichnet, daß sie folgende Zusammensetzung aufweist:

| | |
|---|---|
| Toluidinblau oder metachromatisches analoges Färbemittel | 2,2 g/l |
| Aluminiumsulfat | 7,5 g/l |
| Cetylpyridiniumhalogenid | 0,75 g/l |
| Glutaraldehyd | 20 g/l |
| Formaldehyd | 35 g/l |
| absolutes Methanol | 300 ml/l |
| absolutes Ethanol | 300 ml/l |
| Waser q.s.p. | 1000 ml |

3. Reagens zur gleichzeitigen raschen Fixierung und Färbung, notwendig zur Durchführung des Diagnoseverfahrens nach Anspruch 1, dadurch gekennzeichnet, daß es folgende Zusammensetzung aufweist:

| | |
|---|---|
| Toluidinblau oder analoges metachromatisches Färbemittel | 0,2 bis 0,8 % |
| Aluminiumsulfat | 3 bis 10 % |
| Cetylpyridiniumhalogenid | 0,3 bis 0,8 % |
| Glutaraldehyd | 1,5 bis 2,5 % |
| 30 % Formaldehyd | 270 bis 330 ml |
| Methanol | 270 bis 330 ml |
| Ethanol | 270 bis 330 ml |
| Wasser q.s.p. | 1000 ml |

4. Reagens zur gleichzeitigen raschen Fixierung und Färbung, notwendig zur Durchführung des Diagnoseverfahrens nach Anspruch 1, dadurch gekennzeichnet, daß es folgende Zusammensetzung aufweist:

| | |
|---|---|
| Toluidinblau oder analoges metachromatisches Färbemittel | 0,2 bis 0,8 % |
| Aluminiumsulfat | 3 bis 10 % |
| Cetylpyridiniumhalogenid | 0,3 bis 0,8 % |
| Methanol | 300 ml±10 % |
| 30 % Formaldehyd | 300 ml±10 % |
| Ethanol | 300 ml±10 % |
| Wasser q.s.p. | 1000 ml |

**0 022 698**

5. Reagens nach Anspruch 1, dadurch gekennzeichnet, daß es folgende Zusammensetzung aufweist:

| | |
|---|---|
| Toluidinblau oder analoges metachromatisches Färbemittel | 0,2 bis 0,8 % |
| Aluminiumsulfat | 3 bis 10 % |
| Cetylpyridiniumhalogenid | 0,3 bis 0,8 % |
| Methanol | 830 bis 990 ml |
| Wasser q.s.p. | 1000 ml |

6. Reagens zur gleichzeitigen raschen Fixierung und Färbung, notwendig zur Durchführung des Diagnoseverfahrens nach Anspruch 1, dadurch gekennzeichnet, daß es folgende Zusammensetzung aufweist:

| | |
|---|---|
| Toluidinblau oder analoges metachromatisches Färbemittel | 0,2 bis 0,8 % |
| Aluminiumsulfat | 3 bis 10 % |
| Cetylpyridiniumhalogenid | 0,3 bis 0,8 % |
| Propylenglykol | 250 ml bis 600 ml±10 % |
| Methanol | 200 ml bis 750 ml±10 % |
| Wasser q.s.p. | 1000 ml |

7. Reagens nach einem der Ansprüche 2 bis 6, dadurch gekennzeichnet, daß das eingesetzte Cetylpyridiniumhalogenid das Cetylpyridinium-chlorid oder -bromid ist.

8. Anordnung, geeignet zur Anwendung, oder Kit, vorzugsweise enthalten in einem einzigen Köfferchen, zur Durchführung des diagnostischen Verfahrens für Parasitosen und Allergien nach Anspruch 1, dadurch gekennzeichnet, daß es kombiniert umfaßt:

— mehrere Platten oder Kästen, die jeweils eine bestimmte Anzahl von Vertiefungen gleichen Durchmessers aufweisen, von denen der Hauptanteil, mit Ausnahme der Vertiefungen, die die Kontrollen bilden sollen, eine vorbestimmte Menge eines für eine zu diagnostizierende Parasitose oder Allergie spezifischen Antigensenthält, wobei das Antigen in trockenem Zustand, vorzugsweise lyophilisiert, in jede Vertiefung eingefüllt ist, wobei die genannten vorbestimmten Mengen von einer Vertiefungsreihe zur folgenden varriert und die Vertiefungen bestimmt sind zur Durchführung des diagnostischen Tests nach Anspruch 1, eine geeignete Menge an diagnostischem Reagens für eine Parasitose oder eine Allergie aufzunehmen;

— mehrere Röhrchen, die vordosierte Mengen eines geeigneten Puffers, wie Hepes CM- oder Pipes-Puffer enthalten; mehrere geeignete Behälter, wie Ampullen, Röhrchen oder Spritzen, die jeweils eine Flüssigkeit mit einer Dichte von 1,079—1,085 enthalten;

— mindestens einen Behälter, der eine wässrig-alkoholische Lösung zur gleichzeitigen raschen Fixierung und Färbung der Platten, Kästen oder Analogen, wie vorstehend erwähnt, gemäß einem der Ansprüche 2 bis 7, enthält.

9. Anordnung geeignet zur Verwendung, oder Kit, nach Anspruch 8, dadurch gekennzeichnet, daß:

— jede der Vertiefungen, die ein spezifisches Antigen für die zu untersuchende Parasitose oder Allergie enthält, 10 bis 20 µl des Antigens in lyophilisiertem Zustand enthält und geeignet ist, bei der Durchführung des diagnostischen Tests nach Anspruch 1 variable anwachsende Mengen zwischen 3 und 20 µl des diagnostischen Reagens aufzunehmen;

— wobei jedes Röhrchen, das eine vorbestimmte Puffermenge enthält, 5 ml des Puffers enthält; wobei jeder geeignete Behälter, wie Röhrchen, Ampulle oder Spritze, das die Flüssigkeit mit der Dichte enthält, 5 ml der Flüssigkeit mit einer Dichte von 1,079—1,085 enthält;

— wobei jeder Behälter, der die wässrig-alkoholische Lösung zur gleichzeitigen raschen Fixierung und Färbung, präparierte Platten oder Kästen enthält, eine Lösung mit einem pH-Wert unter 2,5, der die Zusammensetzung nach Anspruch 2 aufweist, enthält.

10. Kit oder Anordnung, geeignet zur Verwendung bei der Durchführung des diagnostischen Tests für Parasitosen und Allergien gemäß Anspruch 1, dadurch gekennzeichnet, daß der Hepes-Puffer, der in vordosierten Mengen in den Ampullen oder geeigneten Röhrchen enthälten ist, 4 bis 25 ml einer molaren

25

Lösung des Hepes-Puffers, 0,932 ml KCl von 10 %, 0,550 ml $CaCl_2$ von 10 %, 0,510 ml $MgCl_2$ von 10 %, 850 bis 950 ml NaCl von 9 ‰, gelöst in einer ausreichenden Wassermenge, um sie auf 1000 ml zu bringen, umfaßt, und/oder, daß die Lösung für die gleichzeitige Fixierung und Färbung der Antigenpräparate (oder Kontrollen), die von den Platten für die diagnostische Ablesung getragen werden, die in vorbestimmten Mengen in Ampullen oder geeigneten einheitlichen Röhrchen enthalten ist, die Zusammensetzung nach einem der Ansprüche 2 bis 7 aufweist.

11. Kit oder Anordnung, geeignet zur Verwendung nach Anspruch 10, dadurch gekennzeichnet, daß er bzw. sie aus einem Köfferchen besteht, das umfaßt:

— mehrere geeignete Behälter, wie Ampullen oder Röhrchen, die vordosierte Mengen eines geeigneten Hepes-Puffers und insbesondere eines Hepes-Pkffers enthaltend 4 bis 0,25 ml einer molaren Lösung von Hepes-Puffer, 0,932 ml KCl von 10 %, 0,550 ml $CaCl_2$ von 10 %, 0,510 ml $MgCl_2$ von 10 %, 850 bis 900 ml NaCl von 9 ‰, in einer ausreichenden Wassermenge, um auf 1000 ml zu bringen, enthält, wobei diese Lösung einen pH-Wert von 7,4—7,6 hat,

— mehrere Ampullen, Röhrchen oder Spritzen, die vordosierte Mengen einer Flüssigkeit mit einer Dichte von 1,079—1,085 enthalten,

— mehrere Platten oder Kästen zur diagnostischen Ablesung, wobei jede bzw. jeder eine bestimmte Anzahl von Vertiefungen aufweist, deren Hauptteil, mit Ausnahme der Kontrollvertiefungen, bestimmt ist zur Aufnahme von vorbestimmten variablen Mengen eines Antigens, das spezifisch für eine zu diagnostizierende Allergie oder Parasitose ist, sowie vorbestimmte variable Mengen des diagnostischen Reagens,

— und mehrere Ampullen oder Röhrchen, die vordosierte Mengen einer Lösung zur gleichzeitigen raschen Fixierung und Färbung enthalten, gemäß einem der Ansprüche 2 bis 7.

12. Kit oder Anordnung, geeignet zur Verwendung nach Anspruch 10, dadurch gekennzeichnet, daß er bzw. sie zwei getrennte Köfferchen umfaßt, von denen einer bestimmt ist zur Abtrennung von basophilen Zellen, und umfaßt:

— mehrere geeignete Behälter, wie Ampullen oder Röhrchen, die vordosierte Mengen eines geeigneten Puffers und insbesondere von Hepes-Puffer gemäß Anspruch 11 enthalten,

— mehrere Ampullen oder Röhrchen, die jeweils vordosierte Mengen der Flüssigkeit mit einer Dichte von 1,079—1,085 enthalten; und wobei das andere Köfferchen enthält:

— mehrere Platten oder Kästen zur diagnostischen Ablesung, die jeweils eine bestimmte Anzahl von Vertiefungen aufweisen, deren Hauptteil, mit Ausnahme der Kontrollvertiefungen, bestimmt ist zur Aufnahme von vorbestimmten variablen Mengen eines Antigens, das spezifisch für eine zu diagnostizierende Allergie oder Parasitose ist, sowie vorbestimmte variable Mengen des diagnostischen Reagens,

— und mehrere Ampullen oder Röhrchen, die vordosierte Mengen einer Lösung zur gleichzeitigen raschen Fixierung und Färbung enthälten, des Typs von Lösungen gemäß einem der Ansprüche 2 bis 7, zur Durchführung des diagnostischen Tests nach Anspruch 1.

13. Kit oder Anordnung, geeignet zur Verwendung nach Anspruch 10, in einem oder zwei Köfferchen, dadurch gekennzeichnet, daß der in vordosierten Mengen in mehreren geeigneten Behältern, wie Röhrchen oder Ampullen, enthaltene Hepes-Puffer aus einer wässrigen Lösung besteht, die 4 bis 25 ml einer molaren Lösung von Hepes-Puffer, 0,932 ml KCl von 10 %, 0,550 ml $CaCl_2$ von 10 %, 0,510 ml $MgCl_2$ von 10 %, 850 bis 900 ml NaCl von 9 ‰, 10 bis 15 Einheiten/ml Heparin, enthält, wobei die Bestandteile in einer Wassermenge gelöst sind, die dazu geeignet ist, die Lösung auf 1000 ml zu bringen, wobei die Lösung einen pH-Wert von 7,4 bis 7,6 aufweist.

14. Kit oder Anordnung, geeignet zur Verwendung nach Anspruch 10, in einem oder zwei Köfferchen, dadurch gekennzeichnet, daß der in vordosierten Mengen in mehreren geeigneten Behältern, wie Röhrchen oder Ampullen, enthaltene Hepes-Puffer, aus einer wässrigen Lösung besteht, die 4 bis 25 ml einer mloaren Lösung von Hepes-Puffer, 0,932 bis 1,2 ml KCl von 10 %, 850 bis 900 ml NaCl von 9 ‰, enthält, wobei die Bestandteile in einer ausreichenden Wassermenge gelöst sind, um die Lösung auf 1000 ml zu bringen, wobei diese Lösung einen pH-Wert von 7,4 bis 7,6 aufweist, und daß die Platten oder Kästen zur diagnostischen Ablesung in jeder der Vertiefungen, die zur Aufnahme des Antigens bestimmt sind, eine vorbestimmte Menge an Calcium und Magnesium in Form ihrer Salze und insbesondere ihrer Chloride enthalten.

15. Kit oder Anordnung, geeignet zur Verwendung nach Anspruch 14, dadurch gekennzeichnet, daß das Calcium auf den Platten oder Kästen zur diagnostischen Ablesung in einer Menge von etwa 1 mMol/l vor handen ist und das Magnesium in einer Menge von etwa 1 mMol/l vorhanden ist.

16. Diagnostisches Reagens zur Durchführung des diagnostischen Tests nach Anspruch 1, insbesondere mit einem Kit nach einem der Ansprüche 9 bis 15, dadurch gekennzeichnet, daß das Reagens aus einer Suspension, angereichert mit polynuklearen Basophilen, die Träger von spezifischen IgE sind, besteht, die 300 bis 1000 Basophile pro µl enthält, und die erhalten wurde aus einer Blutprobe, die einem menschlichen oder tierischen Pateinten entnommen wurde, von dem angenommen wird, daß er von der zu

26

**0 022 698**

diagnotsizierenden Parasitose oder Allergie befallen ist, wobei die Blutprobe der einen oder anderen der folgenden Behandlungen unterworfen wurde:

— entweder wird die Blutprobe einer Zentrifugierung unterworfen während einer geeigneten Dauer und Geschwindigkeit, um eine Leukozytenschicht und ein überstehendes mit Plättchen angereichertes Plasma zu gewinnen, welches verworfen wurde, wobei die Leukozytenschicht gesammelt und mit einer geeigneten Menge eines Puffers vermischt wurde, der auf die basophilen Zellen nicht zerstörend wirkt, wie insbesondere Hepes- CM-Puffer oder Pipes-Puffer;

— oder wird die gesamte Blutprobe mit einem Salz der Ethylendiamintetraessigsäure vermischt und anschliessend mit einem Hepes-Puffer geeigneter Zusammensetzung vermischt;

— oder wird die gesamte Blutprobe mit Ethylendiamintetraessigsäure, vorteilhaft in der Form eines ihrer Salze, und bevorzugt in der Form des Kaliumsalzes (EDTA $K_3$) vermischt und anschließend mit einem Hepes-Puffer geeigneter Zusammensetzung, der Heparin zu 5—20 Einheiten/ml enthält, vermischt;

— worauf eine Flüssigkeit mit einer Dichte von 1,079—1,085, deren Osmolarität bei 280 bis 320 Milliosmol liegt, die vorteilhaft aus einer Zusammensetzung besteht, die Natriummetrizoat und Ficoll oder Percol enthält, unter das Gemisch der Leukozytenschicht und des Puffers oder unter das Gemisch des gesamten über dem Salz der Ethylendiamintetraessigsäure entnommenen Blutes mit einem gegebenenfalls heparinisierten Puffer, injiziert wird,

— das erhaltene Gemisch mit der Flüssigkeit mit der Dicht zentrifugiert wird, um einen Ring zu erhalten, der eine erhöhte Konzentration an Basophilen enthält,

— der Ring entnommen wird und erneut in einem Puffer,. wie dem vorstehend genannten Puffer, suspendiert wird,

— wobei die so gebildete Suspension bereit ist, in die Vertiefungen einer Platte oder eines Kastens zur diagnostischen Ablesung eingebracht zu werden.

17. Reagens nach Anspruch 16, dadurch gekennzeichnet, daß der für die basophilen Zellen nicht zerstörend wirkende Puffer, zu dem die nach dem Zentrifugieren erhaltene Leukozytenschicht gefüht wird, und in dem der mit Basophilen angereicherte Ring, der nach dem Zentrifugieren des vorstehenden Gemischs suspendiert wird, worunter die Flüssigkeit mit der Dichte injiziert wurde, ein Puffer ist, der folgende Zusammensetzung aufweist:

| | |
|---|---|
| Hepes-Puffer, molare Lösung | 25 ml |
| KCl 10 % | 0,932 ml |
| $CaCl_2$ 10 % | 0,550 ml |
| $MgCl_2$ 10 % | 0,510 ml |
| NaCl 9 % (7,6 g) | 844,11 ml |
| $H_2O$ q.s.p. | 1000 ml |

18. Reagens nach Anspruch 17, dadurch gekennzeichnet, daß der Puffer, der nicht zerstörend auf die basophilen Zellen wirkt, zu dem die nach dem Zentrifugieren erhaltene Leukozytenschicht gemischt wurde, und in dem der Ring suspendiert wird, der nach dem Zentrifugieren des vorstehenden Gemischs erhalten wurde, worunter die Flüssigkeit mit der Dichte injiziert wurde, ein Puffer ist, der folgende Zusammensetzung aufweist:

| | |
|---|---|
| Hepes-Puffer, molare Lösung | 4 bis 25 ml |
| KCl 10 % | 0,932 ml |
| $CaCl_2$ 10 % | 0,550 ml |
| $MgCl_2$ 10 % | 0,510 ml |
| NaCl 9 % | 850 bis 900 ml |
| Wasser q.s.p. | 1000 ml |
| pH | 7,4 bis 7,6 |

19. Diagnostisches Reagens nach Anspruch 16, dadurch gekennzeichnet, daß der Puffer, zu dem die

27 ·

# 0 022 698

gesamte Blutprobe gemischt wird, die über einem Ethylendiamintetraessigsäuresalz entnommen wurde, und gegebenenfalls der Puffer, in dem der mit Basophilen angereicherte Ring suspendiert wird, der aus dem Zentrifugieren resultiert, folgende Zusammensetzung aufweist:

| | |
|---|---|
| Hepes-Puffer, molare Lösung | 4 bis 25 ml |
| KCl 10 % | 0,932 ml |
| $CaCl_2$ 10 % | 0,550 ml |
| $MgCl_2$ 10 % | 0,510 ml |
| NaCl 9 ‰ | 850 bis 900 ml |
| Heparin | 10 bis 15 Einheiten/ml |
| Wasser q.s.p. | 1000 ml |
| pH | 7,4 bis 7,6 |

20. Diagnostisches Reagens nach Anspruch 16, dadurch gekennzeichnet, daß der Puffer, in den die gesamte Blutprobe gemischt wird, die über einem Ethylendiamintetraessigsäuresalz entnommen wurde, und gegebenenfalls der Puffer, in dem der mit Basophilen angereicherte Ring, der von dem Zentrifugieren resultiert, suspendiert wird, folgende Zusammensetzung aufweist:

| | |
|---|---|
| Hepes-Puffer, molare Lösung | 4 bis 25 ml |
| KCl 10 % | 0,932 bis 1,2 ml |
| NaCl 9 ‰ | 850 bis 900 ml |
| Wasser q.s.p. | 1000 ml |
| pH | 7,4 bis 7,6 |

mit der Bedingung, daß das benötigte Calcium und Magnesium in der Form ihrer Salze in vorbestimmten Mengen auf den Platten oder Kästen zur diagnostischen Ablesung gemäß dem Verfahren von Anspruch 1, aufgebracht sind.

## Claims

1. A process for detecting parasitoses and allergies, in particular parasitoses due to worms and allergies which cause an increase in the circulating specific immunoglobulins, especially the IgE's, which consists in:— bringing a reagent for detecting parasitoses and allergies, consisting of a suspension of basophilic polynuclear cells carrying specific IgE's, in a suitable buffer, into contact, for about 15 minutes at 37°C, with antigen specific for the parasitosis or allergy which it is desired to detect, contained at different concentrations in a number of wells in a detection slide or dish, while the remaining wells in the said slide or dish contain the detection reagent by itself so as to serve as references; — rapidly drying the slide or dish prepared in this way; — rapidly fixing and staining the slide prepared in this way, using rapid fixing and staining means which selectively stain the cytoplasmic granules of the basophiles; — counting the basophilic polynuclear cells (BP), under an optical microscope at a suitable magnification, respectively in the reference wells and in the different wells containing the reagent and different concentrations of antigen; — and establishing the degranulation index:

$$DI = \frac{BP\ reference - BP\ antigen}{BP\ reference} \times 100$$

or percentage of basophiles which has apparently disappeared in the presence of the antigen, by comparison of the decrease in the number of basophilic polynuclear cells in the wells containing the antigen at various concentrations with the number of basophilic polynuclear cells contained in the reference wells, which index, if greater than 35%, makes it possible to detect the presence of the parasitosis or allergy sought, the said process being characterized in that the detection reagent introduced into the wells in the abovementioned slide or dish consists of a suspension enriched in basophilic polynuclear cells in a buffer which is non-destructive towards the basophilic cells, such as Hepes CM buffer or Pipes buffer in

28

particular, which suspension contains from 300 to 1000 basophiles per µl and has been obtained from a blood sample taken from a human or animal patient supposedly affected by the parasitosis or allergy to be detected, which blood sample has been subjected to one or other of the following treatments:

— the blood sample is subjected to centrifugation, for an appropriate time and at an appropriate speed, so as to collect a leukocyte layer and a supernatant plasma rich in platelets, which was discarded while the leukocyte layer collected was mixed with an appropriate quantity of a buffer which is non-destructive towards the basophilic cells, such as Hepes CM buffer or Pipes buffer in particular;

— or the said whole blood sample is mixed with an ethylenediaminetetraacetic acid salt and then mixed with a Hepes buffer of appropriate composition;

— or the said whole blood sample is mixed with ethylenediaminetetraacetic acid, advantageously in the form of one of its salts and preferably in the form of its potassium salt (EDTA $K_3$), and then mixed with a Hepes buffer of appropriate composition, containing 5—20 units of heparin per ml;

— after which a liquid of specific gravity 1.079—1.085, whose osmolarity is between 280 and 320 milliosmol and which advantageously consists of a composition comprising sodium metrizoate and Ficoll or Percol, is injected under the mixture of leukocyte layer and buffer or under the mixture of whole blood sample, taken on an ethylenediaminetetraacetic acid salt, with a buffer optionally containing heparin;

— the mixture obtained being centrifuged with the said liquid of specific gravity 1.079—1.085 to give a ring containing a high concentration of basophiles;

— which ring is removed and then resuspended in a buffer such as the buffer mentioned above;

— the said process also being characterized in that the abovementioned rapid fixing and staining means consist of a single composition which simultaneously and rapidly fixes and stains and which comprises a metachromatic dye, preferably toluidine blue, associated with aluminium sulphate, a cetylpyridinium halide and

— glutaraldehyde and formaldehyde, dissolved in a mixture of absolute ethanol, absolute methanol and water, which solution has an acid pH below 2.5;

— or methanol optionally associated with formaldehyde and/or ethanol, in the absence of glutaraldehyde;

— or propylene glycol associated with methanol, in the absence of glutaraldehyde, formaldehyde and ethanol.

2. As a reagent for simultaneous and rapid fixing and staining, necessary for carrying out the detection process according to Claim 1, an aqueous-alcoholic solution with an acid pH below 2.5, characterized in that it has the following composition:

| | |
|---|---|
| Toluidine blue or analogous metachromatic dye | 2.2 g/litre |
| Aluminium sulphate | 7.5 g/litre |
| Cetylpyridinium halide | 0.75 g/litre |
| Glutaraldehyde | 20 g/litre |
| Formaldehyde | 35 g/litre |
| Absolute methanol | 300 ml/litre |
| Absolute ethanol | 300 ml/litre |
| Water q.s. | 1000 ml |

# 0 022 698

3. A reagent for simultaneous and rapid fixing and staining, necessary for carrying out the detection process according to Claim 1, characterized in that it has the following composition:

| | |
|---|---|
| Toluidine blue or analogous metachromatic dye | 0.2 to 0.8 % |
| Aluminium sulphate | 3 to 10 % |
| Cetylpyridinium halide | 0.3 to 0.8 % |
| Glutaraldehyde | 1.5 to 2.5 % |
| 30% formaldehyde solution | 270 to 330 ml |
| Methanol | 270 to 330 ml |
| Ethanol | 270 to 330 ml |
| Water          q.s. | 1000 ml |

4. A reagent for simultaneous and rapid fixing and staining, necessary for carrying out the detection process according to Claim 1, characterized in that it has the following composition:

| | |
|---|---|
| Toluidine blue or analogous metachromatic dye | 0.2 to 0.8 % |
| Aluminium sulphate | 3 to 10 % |
| Cetylpyridinium halide | 0.3 to 0.8 % |
| Methanol | 300 ml±10 % |
| 30% formaldehyde solution | 300 ml±10 % |
| Ethanol | 300 ml±10 % |
| Water          q.s. | 1000 ml |

5. A reagent according to Claim 1, characterized in that it has the following composition:

| | |
|---|---|
| Toluidine blue or analogous metachromatic dye | 0.2 to 0.8 % |
| Aluminium sulphate | 3 to 10 % |
| Cetylpyridinium halide | 0.3 to 0.8 % |
| Methanol | 830 to 990 ml |
| Water          q.s. | 1000 ml |

6. A reagent for simultaneous and rapid fixing and staining, necessary for carrying out the detection process according to Claim 1, characterized in that it has the following composition:

| | |
|---|---|
| Toluidine blue or analogous metachromatic dye | 0.2 to 0.8 % |
| Aluminium sulphate | 3 to 10 % |
| Cetylpyridinium halide | 0.3 to 0.8 % |
| Propylene glycol | 250 ml to 600 ml±10 % |
| Methanol | 200 ml to 750 ml±10 % |
| Water          q.s. | 1000 ml |

7. A reagent according to any one of Claims 2 to 6, characterized in that the cetylpyridinium halide used is cetylpyridinium chloride or bromide.

30

8. A ready-to-use set, or kit, preferably contained in a single box, for carrying out the test for detecting parasitoses and allergies, according to Claim 1, characterized in that it comprises a combination of the following:— a plurality of slides or dishes each having a number of wells of equal diameter, of which the majority, with the exception of the wells which are to constitute the references, contain a predetermined quantity of an antigen specific for a parasitosis or allergy to be detected, the said antigen being introduced into each of the wells in the dry state, preferably lyophilized, the said predetermined quantities varying fcom one column of wells to the next and the said wells being intended to receive, for the purpose of carrying out the detection test according to Claim 1, an appropriate quantity of a reagent for detecting a parasitosis or allergy; — a plurality of tubes containing previously measured quantities of an appropriate buffer such as Hepes CM or Pipes buffer; — a plurality of suitable receptacles such as ampoules, tubes or syringes, each containing a liquid of specific gravity 1.079—1.085; and at least one receptacle containing an aqueous-alcoholic solution for the simultaneous and rapid fixing and staining of the abovementioned slides, dishes or the like, according to any one of Claims 2 to 7.

9. The ready-to-use set, or kit, according to Claim 8, characterized in that:— each of the wells containing an antigen specific for the parasitosis or allergy to be detected contains 10 to 20 µl of the said antigen in the lyophilized state and is capable of receiving, when the detection test according to Claim 1 is carried out, increasing variable quantities of between 3 and 20 µl of detection reagent; — each tube containing a previously measured quantity of buffer contains 5 ml of the said buffer; — each suitable receptacle such as a tube, ampoule or syringe, containing liquid of specific gravity 1.079—1.085, contains 5 ml of the said liquid; — and the receptacle or each receptacle containing the aqueous-alcoholic solution for the simultaneous and rapid fixing and staining of the prepared slides or dishes contains a solution with a pH below 2.5 and having the composition according to Claim 2.

10. A kit or ready-to-use set for carrying out the test for detecting parasitoses and allergies, according to Claim 1, characterized in that the Hepes buffer contained in previously measured quantities in appropriate ampoules or tubes comprises from 4 to 25 ml of a molar solution of Hepes buffer, 0.932 ml of 10% KCl, 0.550 ml of 10% $CaCl_2$, 0.510 ml of 10% $MgCl_2$ and from 850 to 900 ml of 9‰ NaCl, dissolved in a sufficient quantity of water to make up the volume to 1000 ml, and/or in that the solution for the simultaneous and rapid fixing and staining of the antigen preparations (or references) carried by the detection slides, contained in previously measured quantities in appropriate individual ampoules or tubes, has the composition according to any one of Claims 2 to 7.

11. The kit or ready-to-use set according to Claim 10, characterized in that it is made up of a box which comprises:— a plurality of suitable receptacles such as ampoules or tubes, containing previously measured quantities of an appropriate Hepes buffer, especially a Hepes buffer comprising 4 to 0.25 ml of a molar solution of Hepes buffer, 0.932 ml of 10% KCl, 0.550 ml of 10% $CaCl_2$, 0.510 ml of 10% $MgCl_2$ and 850 to 900 ml of 9‰ NaCl, in a sufficient quantity of water to make up the volume to 1000 ml, this solution having a pH of 7.4—7.6; — a plurality of ampoules, tubes or syringes containing previously measured quantities of a liquid of specific gravity 1.079—1.085; — a plurality of detection slides or dishes each having a number of wells, of which the majority, with the exception of the reference wells, are intended to receive variable predetermined quantities of an antigen specific for an allergy or parasitosis to be detected, as well as variable predetermined quantities of detection reagent; — and a plurality of ampoules or tubes containing previously measured quantities of a solution for simultaneous and rapid fixing and staining, according to any one of Claims 2 to 7.

12. The kit or ready-to-use set according to Claim 10, characterized in that it is made up of two separate boxes, one of which, namely the one intended for separation of the basophilic cells, comprises:— a plurality of suitable receptacles such as ampoules or tubes, containing previously measured quantities of an appropriate buffer, especially Hepes buffer according to Claim 11; — and a plurality of ampoules or tubes each containing previously measured quantities of a liquid of specific gravity 1.079—1.085; and the other of which comprises:— a plurality of detection slides or dishes each having a number of wells, of which the majority, with the exception of the reference wells, are intended to receive variable predetermined quantities of an antigen specific for an allergy or parasitosis to be detected, as well as variable predetermined quantities of detection reagent; — and a plurality of ampoules or tubes containing previously measured quantities of a solution for simultaneous and rapid fixing and staining, of the same type as the solutions according to any one of Claims 2 to 7, for carrying out the detection test according to Claim 1.

13. The kit or ready-to-use set according to Claim 10, in one or two boxes, characterized in that the Hepes buffer contained in previously measured quantities in a plurality of suitable receptacles such as tubes or ampoules consists of an aqueous solution comprising 4 to 25 ml of a molar solution of Hepes buffer, 0.932 ml of 10% KCl, 0.550 ml of 10% $CaCl_2$, 0.510 ml of 10% $MgCl_2$, 850 to 900 ml of 9‰ NaCl and 10 to 15 units of heparin per ml, which constituents are dissolved in a sufficient quantity of water to make up the solution to 1000 ml, this solution having a pH of between 7.4 and 7.6.

14. The kit or ready-to-use set according to Claim 10, in one or two boxes, characterized in that the Hepes buffer contained in previously measured quantities in a plurality of suitable receptacles such as tubes or ampoules consists of an aqueous solution comprising 4 to 25 ml of a molar solution of Hepes buffer, 0.932 to 1.2 ml of 10% KCl and 850 to 900 ml of 9‰ NaCl, which constituents are dissolved in a sufficient quantity of water to make up the solution to 1000 ml, this solution having a pH of between 7.4 and

31

7.6, and in that the detection slides or dishes carry, in each of their wells intended to receive antigen, a predetermined quantity of calcium and magnesium in the form of their salts, especially their chlorides.

15. The kit or ready-to-use set according to Claim 14, characterized in that the calcium is present on the detection slides or dishes at a concentration of about 1 mmol/litre and the magnesium is present at a concentration of about 1 mmol/litre.

16. A detection reagent for carrying out the detection test according to Claim 1, in particular using the kit according to any one of Claims 9 to 15, characterized in that the said reagent consists of a suspension enriched in basophilic polynuclear cells carrying specific IgE's, which contains from 300 to 1000 basophiles per µl and which as been obtained from a blood sample taken from a human or animal patient supposedly affected by the parasitosis or allergy to be detected, which blood sample has been subjected to one or other of the following treatments:

— the blood sample is subjected to centrifugation, for an appropriate time and at an appropriate speed, so as to collect a leukocyte layer and a supernatant plasma rich in platelets, which was discarded while the leukocyte layer collected was mixed with an appropriate quantity of a buffer which is non-destructive towards the basophilic cells, such as Hepes CM buffer or Pipes buffer in particular;
— or the said whole blood sample is mixed with an ethylenediaminetetraacetic acid salt and then mixed with a Hepes buffer of appropriate composition;
— or the said whole blood sample is mixed with ethylenediaminetetraacetic acid, advantageously in the form of one of its salts and preferably in the form of its potassium salt (EDTA $K_3$), and then mixed with a Hepes buffer of appropriate composition, containing 5—20 units of heparin per ml;
— after which a liquid of specific gravity 1.079—1.085, whose osmolarity is between 280 and 320 milliosmol and which advantageously consists of a composition comprising sodium metrizoate and Ficoll or Percol, is injected under the mixture of leukocyte layer and buffer or under the mixture of whole blood sample, taken on an ethylenediaminetetraacetic acid salt, with a buffer optionally containing heparin;
— the mixture obtained being centrifuged with the said liquid of specific gravity 1.079—1.085 to give a ring containing a high concentration of basophiles;
— which ring is removed and then resuspended in a buffer such as the buffer mentioned above;
— the suspension thus formed being ready to be deposited in the wells in a detection slide or dish.

17. A reagent according to Claim 16, characterized in that the buffer which is non-destructive towards the basophilic cells, with which the leukocyte layer obtained after centrifugation is mixed, and in which the ring rich in basophiles, obtained after centrifugation of the above mixture under which the said liquid of specific gravity 1.079—1.085 was injected, is suspended, is a buffer having the following composition:

| | |
|---|---|
| Hepes buffer, molar solution | 25 ml |
| KCl, 10 % | 0.932 ml |
| $CaCl_2$, 10 % | 0.550 ml |
| $MgCl_2$, 10 % | 0.510 ml |
| NaCl, 9 ‰ (7.6 g) | 844.11 ml |
| $H_2O$ q.s. | 1000 ml |

18. A reagent according to Claim 17, characterized in that the buffer which is non-destructive towards the basophilic cells, with which the leukocyte layer obtained after centrifugation is mixed, and in which the ring rich in basophiles, obtained after centrifugation of the above mixture under which the said liquid of specific gravity 1.079—1.085 was injected, is suspended, is a buffer having the following composition:

| | |
|---|---|
| Hepes buffer, molar solution | 4 to 25 ml |
| KCl, 10 % | 0.932 ml |
| $CaCl_2$, 10 % | 0.550 ml |
| $MgCl_2$, 10 % | 0.510 ml |
| NaCl, 9 ‰ | 850 to 900 ml |
| Water q.s. | 1000 ml |
| pH | 7.4—7.6 |

32

19. A detection reagent according to Claim 16, characterized in that the buffer with which the whole blood sample taken on an ethylenediaminetetraacetic acid salt is mixed, and, if appropriate, the buffer in which the ring rich in basophiles, resulting from centrifugation, is suspended, has the following composition:

| | |
|---|---|
| Hepes buffer, molar solution | 4 to 25 ml |
| KCl, 10 % | 0.932 ml |
| $CaCl_2$, 10 % | 0.550 ml |
| $MgCl_2$, 10 % | 0.510 ml |
| NaCl, .9 ‰ | 850 to 900 ml |
| Heparin | 10 to 15 units/ml |
| Water          q.s. | 1000 ml |
| pH | 7.4—7.6 |

20. A detection reagent according to Claim 16, characterized in that the buffer in which the whole blood sample taken on an ethylenediaminetetraacetic acid salt is mixed, and, if appropriate, the buffer in which the ring rich in basophiles, resulting from centrifugation, is suspended, has the following composition:

| | |
|---|---|
| Hepes buffer, molar solution | 4 to 25 ml |
| KCl, 10 % | 0.932 to 1.2 ml |
| NaCl, 9 ‰ | 850 to 900 ml |
| Water          q.s. | 1000 ml |
| pH | 7.4—7.6, |

with the proviso that the necessary calcium and magnesium are deposited in the form of their salts, in predetermined quantities, on the detection slides or dishes, according to the process forming the subject of Claim 1.

33

# Fig. 1

(a)

(b)

Fig. 2

Fig. 3

0 022 698